# EUROPEAN PATENT APPLICATION

(11) **EP 3 444 358 A1**
(43) Date of publication of application: **20.02.2019**
(21) Application number: 18178491.9
(22) Date of filing: 12.02.2010
(51) Int. Cl.: C12Q 1/68, G01N 33/68, G01N 33/574

(54) **METHODS FOR DIAGNOSIS AND PROGNOSIS OF COLORECTAL CANCER**

(30) Priority: 20.02.2009 US 154303 P
(62) Divisional of application: 10744046.3
(71) Applicant: Onconome, Inc., Redmond, WA 98052 (US)
(72) Inventor: AW, Eva, I-wei, Shoreline, WA 98052 (US)
(74) Representative: Schiweck Weinzierl Koch Patentanwälte Partnerschaft mbB

(57) **Abstract**

The present invention provide methods and compositions related to the detection of colorectal cancer based upon the identification of biomarkers and combinations of biomarkers that indicate the present of colorectal cancer. One embodiment of the present invention provides a method for detecting colorectal cancer in a subject by obtaining a biological sample from the subject; detecting one or more biomarkers present in the sample; and comparing the concentrations and/or expression levels of the one or more biomarkers within the biological sample with the concentrations and/or expression levels of the one or more biomarkers in a normal control sample.

## Description

### Cross Reference to Related Application

This application claims the benefit of Provisional Application No. 61/154,303, filed February 20, 2009.

### Technical Field

The present invention relates to methods of diagnosing colorectal cancer and, in particular, to the use of a panel of biomarkers in the diagnosis of colorectal cancer from a biological sample.

### Background

Colorectal cancer (CRC) is the third most prevalent malignancy in the United States with approximately 145,000 new diagnoses and 56,000 deaths estimated for 2005. Despite advances made, the efficacy of therapy has reached a plateau, making early diagnosis fundamental to reduce morbidity and mortality, especially in view of the fact that patients diagnosed at early stages show long-term survival. Stage I patients have a survival rate of ∼85%, while the 5-year survival rate drops to ∼65-75% in stage II patients and to 35-50% in stage III patients.

The most common non-invasive test for colorectal cancer is the fecal occult blood test ("FOBT"). Unfortunately, in addition to its high false-positive rate, the sensitivity of the FOBT remains around 50% and may not detect early malignancy, since not all carcinomas shed blood. Numerous serum markers, such as carcinoembryonic antigen ("CEA"), carbohydrate antigen 19-9, and lipid-associated sialic acid, have been investigated in colorectal cancer, but their low sensitivity has induced the American Society of Clinical Oncology to state that none can be recommended for screening and diagnosis, and that their use should be limited to postsurgery surveillance. Colonoscopy and sigmoidoscopy remain the gold standard for detecting colon cancer. These invasive exams are expensive, require highly trained staff, are uncomfortable, and raise the risk of bowel perforation and possible mortality. In addition, the normal sterilization process for endoscope, while effective against bacteria and many viruses, may not be effective against prions, and thus colonoscopy potentially expose patients to prion infection. Consequently, there is still a great need for new biomarkers and diagnostic tests for colorectal cancer. Since the treatment for colorectal cancer is very much stage-dependent, clinicians, researchers, and various additional medical personnel and, ultimately, medical patients, all continue to seek a diagnostic tool for colorectal-cancer-stage identification.

### Summary

Certain embodiments of the present invention provide methods and compositions related to the detection of colorectal cancer based upon the identification of biomarkers and combinations of biomarkers that indicate the present of colorectal cancer. One embodiment of the present invention provides a method for detecting colorectal cancer in a subject by obtaining a biological sample from the subject; detecting one or more biomarkers present in the sample; and comparing the concentrations and/or expression levels of the one or more biomarkers within the biological sample with the concentrations and/or expression levels of the one or more biomarkers in a normal control sample.

### Brief Description of the Drawings

The following Detailed Description, given by way of examples, but not intended to limit the invention to specific embodiments described, may be understood in conjunction with the accompanying figures, in which:
Figure 1 shows an SDS-PAGE gel image of 14 serum samples.
Figures 2A and 2B show western blots for colorectal cancer and normal serum samples probed with 1µg/ml rabbit polyclonal anti human fibronectin antibodies and 1µg/ml of rabbit IgG1 isotype antibodies as a negative control.
Figure 3 shows representative selected-reaction-monitoring mass spectrometry ("SRM-MS") chromatograms for α-1-acid glycoprotein 1 ("ORM 1") working peptides.
Figure 4 shows representative multiple-reaction-monitoring mass spectrometry ("MRM-MS") peptide trend lines of three ORM1 working peptides for 33 serum samples.
Figure 5 shows boxplots of cancer vs. normal for serum values for amyloid A protein ("SAA2"), ORM1, plasma serine protease inhibitor ("SERPINA3"), and C9 complement component ("C9").
Figure 6 shows a matrix plot for SERPINA3, ORM1, SAA2, and C9.
Figure 7 shows a hierarchical clustering analysis of plasma and serum samples.
Figure 8 shows an MRM-MS C9 trend line for three transition peptides of the C9 protein.
Figure 9 shows a Receiver Operating Characteristic ("ROC") curve for a 48-serum-sample set using the random-forest model.
Figure 10 shows an ROC curve for 48 serum samples using the boosting method.
Figure 11 shows an ROC curve for a 33-serum-sample set constructed by the random-forest model.
Figure 12 shows an ROC curve for 13-serum-sample set constructed by the random-forest model.

### Detailed Description

Certain embodiments of the present invention are described below, in overview, followed with experimental examples. Two appendixes with sequences listing are provided following the detailed description.

Mass spectrometry-based strategies for protein identification and quantification have made it possible to perform global, large scale comparative proteomic analysis of complex biological samples. Specifically, multiple-reaction-monitoring mass spectrometry ("MRM-MS"), a state-of-art mass spectrometry mode, offers very high sensitivity and speed for the identification and quantification of specific peptides in complex biological mixtures, and thus has promise for high-throughput screening of clinical samples for candidate markers. MRM-MS has been well established in the pharmaceutical industry for small-molecule detection and in clinical laboratories for analysis of drug metabolites. Given the large number of potential biomarkers for disease detection, conventional diagnostic tools such as ELISA, which require expensive reagents and long development times, are not generally suitable for proteomic analysis. A portable MRM-MS assay, which provides low cost and fast turn-around time, is an attractive choice as the next generation assay platform in clinical laboratories, and is a promising basis for developing a diagnostic tool for colorectal-cancer-stage identification.

Certain embodiments of the present invention are based, in part, on the identification of a panel of biomarkers that are associated with colorectal cancer. These biomarkers are listed in Table 1. These biomarkers are present at different levels in the biological samples of colorectal cancer patients than in normal control samples. Accordingly, certain embodiments of the present invention relates to methods for the diagnosis, prognosis, and monitoring of colorectal cancer, including the different stages of colorectal cancer, by detecting or determining, in a biological sample obtained from a subject, the presence of an amount or level of at least one biomarker identified in Table 1. In particular embodiments of the present invention, the presence, an amount, and/or a level of at least two biomarkers, at least three biomarkers, at least four biomarkers, at least five biomarkers, at least six biomarkers, at least 7 biomarker, at least 8 biomarkers, etc. (including at least 9, 10, 11, 12, 13, 14, 15, 16, 17, 18, 19, 20, or more biomarkers, in any combination) of the biomarkers listed in Table 1 are determined. In alternative embodiments of the present invention, the amounts or levels of additional biomarkers, not listed in Table 1, may also be determined, including carcinoembryonic antigen ("CEA"), carbohydrate antigen 19-9, and, in still further embodiments of the present invention, to detect different types of cancer or other pathologies.

The term "biological sample" refers to any biological sample obtained from a human subject, including, e.g., a tissue sample, a cell sample, a tumor sample, and a biological fluid such as blood, serum, plasma, or urine. In one embodiment of the present invention, the biological sample is serum.

A "biomarker" is a molecule produced by a cell or a tissue in an organism whose presence, level of expression, or form is correlated with cancer, e.g., colorectal cancer. Such molecules include nucleic acids, oligonucleotides, polynucleotides, peptides, polypeptides, and proteins, including polynucleotides, peptides, polypeptides, and proteins modified by the addition of polysaccharides, lipids, and various small molecules and functional groups.

### Methods for Diagnosis

In certain embodiments of the present invention, the presence of colorectal cancer in a subject may be detected by a difference between the expression levels of one or more of the selected biomarkers of Table 1 in a biological sample from a patient and the expression levels of the same one or more selected biomarkers in one or more normal control samples. The expression levels of one or more biomarkers may be greater in the biological sample than in the control sample (i.e., an up-regulated biomarker) or may be less in the biological sample than in the control sample (i.e., a down-regulated biomarker). The threshold for classifying a biomarker expression level as up-regulated may be a constant multiplier of the normal-control-sample expression level, between about 1.05 to about 50, depending on the biomarker and the pathology being diagnosed. Similarly, the threshold for classifying a biomarker expression level as down-regulated may be a constant multiplier of the normal-control-sample expression level, between about 0.9 to about 0.1 or less, depending on the biomarker and pathology being diagnosed.

One embodiment of the present invention comprises a method that distinguishes stage I from stage II and stage III colorectal cancer. The method comprising the following steps: (1) determining the level of expression of one or more biomarkers of Table 1 in a biological sample from a subject having colorectal cancer; (2) comparing the level of the expression of the one or more biomarkers in the biological sample of the subject to the level of expression of the same one or more biomarkers in a normal control or to a predetermined control value to determine the degree of change in expression the one or more biomarkers in the subject sample; (3) comparing the degree of change determined for each biomarker in step (2) to predetermined reference values associated with stage I colorectal cancer. When the degree of change is greater than the predetermined reference value of stage I colorectal cancer for one or more up-regulated biomarkers, and/or the fold change is less than the predetermined reference value of stage I colorectal cancer for one or more down-regulated biomarkers, the subject is diagnosed as having stage II or stage III colorectal cancer.

Another embodiment of the invention comprises a method that is used to manage the treatment of colorectal cancer and to monitor the efficacy of colorectal cancer therapy, and to indicate the recurrence of the cancer. The method comprises the following steps: (1) repeatedly determining the level of expression of one or more biomarkers of Table 1 in a biological sample from a subject having colorectal cancer; (2) comparing each of the levels of the expression of the one or more biomarkers in the sample to the levels of expression of the same one or more biomarkers in a normal control group or to a predetermined control value in order to obtain a series of comparisons during the course of a treatment; (3) for each of the one or more biomarkers, when the differences between determined levels of the one or more biomarkers and the corresponding control values decrease, determining that the treatment appears to be effective with respect to the one or more biomarkers; and (4) determining an over-all effectiveness of treatment by determining the ratio of biomarkers with respect to which the treatment appears effective to the total number of biomarkers.

One embodiment of the present invention provides a kit for diagnosing colorectal cancer that can detect the expression of the biomarkers in a biological sample. For example, the kit may include reagents suitable for performing an antibody-based immunoassay, such as an enzyme immunoassay ("ELISA"), a radioimmunoassay ("RIA"), and/or an immunohistochemical test. For example, a kit may comprise binding agents (*e.g.,* antibodies) specific for one or more of the biomarker proteins, or fragments, listed in Table 1. In addition, the kit may comprise one or more of standards, assay diluent, wash buffer, and a solid support such as microtiter plates. In another embodiment of the present invention, a kit may comprise reagents suitable for performing a reverse-transcription polymerase chain reaction ("RT-PCR") assay that measures nucleic-acid encoding one or more of the protein biomarkers of Table 1. For example, the kit may comprise one or more of a means for isolating total RNA from a biological sample, a means for generating cDNA from isolated total RNA, and pairs of primers suitable for amplifying nucleic acids encoding one or more of the protein biomarkers listed in Table 1.

### Methods for detecting biomarkers

Certain method embodiments of the present invention may be practiced by determining the expression level of one or more biomarkers using any technique known in the art. Various techniques may be used to detect mRNA and/or protein levels of biomarkers, including those described below. Mass spectrometry methods are well-known in the art and have been used to quantify and/or identify biomolecules such as proteins. In one embodiment of the present invention, one or more markers listed in Table 1 can be detected and analyzed using chromatographic techniques, such as high pressure liquid chromatograph ("HPLC") and gel electrophoresis coupled with mass spectrometry, such as tandem mass spectrometry ("MS/MS"), liquid chromatography tandem mass spectrometry ("LC/MS/MS"), matrix assisted laser desorption ionization time-of-flight mass spectrometry ("MALDI-TOF/MS"), and surface enhanced laser desorption ionization mass spectrometry ("SELDI-MS"). One embodiment of the present invention comprises the following steps: (1) determining the level of expression of one or more biomarkers of Table 1 in a biological sample from a subject having, or suspected of having, colorectal cancer; and (2) comparing the concentrations and/or levels of expression of the biomarkers in the biological sample with the corresponding concentrations and/or levels of expression in a normal control group or with predetermined values. In particular embodiments of the present invention, the presence of colorectal cancer is determined when one or more examined biomarkers are differentially expressed above or below up-regulation and down-regulation thresholds, respectively, in the subject sample as compared to a control or are detected at greater or less than threshold concentrations with respect to predetermined values. However, the presence of colorectal cancer may also be determined when the expression level or concentration of one or more of the biomarkers tested is differentially expressed.

In one embodiment of the present invention, biomarkers listed in Table 1 can be identified, analyzed, and quantified using MRM-MS. Specific tryptic peptides can be selected as stoichiometric representatives of the protein markers from which they are cleaved. The selected tryptic peptides can be quantified against a stable isotope-labeled peptide as an internal standard to provide a measure of the concentration of the protein, or can be quantified relatively by comparing the expression level against a normal control. One specific assay comprises a LC/MS/MS based assay coupled with a relative quantitation MRM-MS.

In another embodiment or the present invention, a biomarker is detected in a biological sample by measuring the biomarker protein using an immunoassay, such as Western blotting analysis or an enzyme-linked immunoabsorbent assay ("ELISA"). A variety of immunoassay methods can be used to measure the biomarker proteins. Antibodies specific to the various biomarkers of Table 1 may be readily obtained or produced using standard techniques.

In yet another embodiment of the present invention, a biomarker in a biological sample is detected by measuring nucleic acid, e.g., mRNA, encoding a protein biomarker of Table 1. In one embodiment of the present invention, the biological sample may be isolated RNA. The detection of RNA transcripts may be achieved by Northern blotting analysis in which a preparation of RNA is run on a denaturing agarose gel and transferred to a suitable support, such as nitrocellulose or nylon membranes. Radiolabeled cDNA or RNA is then hybridized to the preparation, washed, and analyzed by autoradiography. The detection of RNA transcripts may also be achieved by various known amplification methods such as RT-PCR.

### Screening for Therapeutics

Differential expression of biomarkers may be the result of an aberrant expression of the biomarkers at either the genomic (e.g., gene amplification), transcriptomic (e.g., increased mRNA transcription products), or proteomic levels (i.e, translation, post-translational modifications etc.) within a given subject. Aberrant over-expressed biomarkers may be regulated using agents that inhibit their biological activity and/or biological expression, while aberrant under-expressed biomarkers may be regulated using agents that can promote their biological activity or biological expression. Such agents can be used to treat a subject having colorectal cancer, and are referred to as "therapeutic agents". Agents capable of interacting directly or indirectly with a biomarker in Table 1 can be identified by various methods that are known in the art, such as binding assays, including yeast-2-hybrid and phage display. One embodiment of the present invention provides methods for screening therapeutic agents for treating colorectal cancer resulting from aberrant expression of one or more biomarkers listed in Table 1 below:

**Table 1: A list of 20 marker proteins that are expressed differently in a subject having colorectal cancer and a normal control**

| **Protein Name/Gene Name** | **accession no. swiss-prot Gene ID** | **Up or down regulated** | **p-value** |
|---|---|---|---|
| α-1-acid glycoprotein 1 | P02673 | up | 0.042 |
| ORM1 | 5004 | | |
| Gelsolin | P06396 | down | 0.013 |
| GSN | 2934 | | |
| C2 complement | P06681 | up | 0.051 |
| C2 | 717 | | |
| C9 Complement Component | P02748 | up | 0.0082 |
| C9 | 735 | | |
| Pregnancy zone protein | P20742 | up | 0.0094 |
| PZP | 5858 | | |
| C-reactive protein | P02741 | up | 0.0382 |
| CRP | 1401 | | |
| Complement factor H-related protein1 | Q03591 | up | 0.0003 |
| CFHR1 | 3078 | | |
| Plasma serine protease inhibitor | P05154 | up | 0.0108 |
| SERPINA3 | 5104 | | |
| Hyaluronan-binding protein 2 | Q14520 | up | 0.0111 |
| HABP2 | 3026 | | |
| Beta-Ala-His dipeptidase | Q96KN2 | down | 0.0032 |
| CNDP1 | 84735 | | |
| Complement factor H-related protein 2 | P36980 | up | 0.0019 |
| CFHR2 | 3080 | | |
| Serum amyloid A protein | P02735 | up | 0.0069 |
| SAA | 6288,6289 | | |
| LOC653879 similar to complement C3 | P01024 | up | 0.0109 |
| LOC653879 | 653879 | | |
| Vitamin K-dependent protein Z | P22891 | down | 0.0029 |
| PROZ | 8858 | | |
| Serum paraoxonase/lactonase 3 | Q15166 | down | 0.0305 |
| PON3 | 5446 | | |
| Retinoic acid receptor responder protein 2 | Q99969 | up | 0.0023 |
| RARRES2 | 5919 | | |
| Gamma-glutamyl hydrolase | Q92820 | up | 0.0041 |
| GGH | 8836 | | |
| proteoglycan-4 | Q92954 | up | 0.0202 |
| PRG4 | 10216 | | |
| Cell surface glycoprotein MUC18 | P43121 | up | 0.006 |
| MCAM | 4162 | | |
| FN1 Isoform 8 of fibronectin | P02751 | up | 0.024 |
| FN1 | 2335 | | |

It should be appreciated that the present invention should not be limited to the biomarkers listed above in Table 1. Additional biomarkers maybe discovered to detect colorectal cancer and other types of cancer or other pathologies. **Appendix 1:** provides amino acid sequence for the 20 biomarkers in Table 1. **Appendix 2:** provides nucleotide sequence for the 20 biomarkers in Table 1.

### Examples

The present invention should not be construed to be limited to the examples described here. Embodiments of the present invention include any and all applications provided and all equivalent variations within the skill of the ordinary artisan.

### Example 1: Identification of biomarkers for colorectal cancer: discovery phase

### Test serum samples

A total of 14 serum samples were examined, including 8 colorectal cancers: 2 of stage I, 2 of stage II, 2 of stage III, and 2 of stage IV, as well as 6 normal age and gender matched controls.

### Gel-Enchanced LC/MS/MS

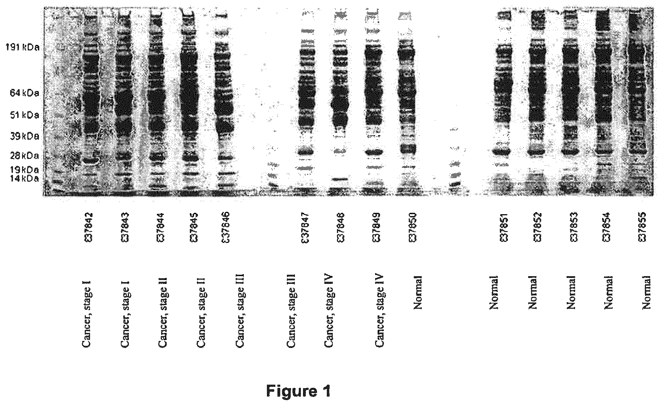

Using the MARS-7 spin column (Agilent), 2 µL of each serum sample was depleted and protein was quantified post-depletion. SDS-PAGE loading buffer was used to solublize 20 µg of each sample. A 4-12% Bis-Tris Novex gel (Invitrogen) was run for each sample in singlet. Figure 1 shows an SDS-PAGE gel image of 14 serum samples. Each lane 101 to 114 was loaded with 20 µg of each serum sample. The gel was stained with coomassie (SimplyBlue) and then excised into 24 bands per lane using a grid.

### In-gel digestion

Each band was subjected to trypsin digestion using a ProGuest workstation as follows: (1) samples were reduced with DTT at 60°C and allowed to cool to room temperature; (2) samples were alkylated with iodoacetamide and incubated at 37°C for 4 hours in the presence trypsin; (3) formic acid was added to stop the reaction.

### LC/MS/MS

Gel digests were analyzed using nano LC/MS/MS on a Thermo LTQ Orbitrap XL. 30µl of hydrolysate were loaded on a 75µm C12 vented column at a flow-rate of 10µL/min and eluted at 300nL/min. A one hour gradient was employed. Product ion data were searched against the IPI Human v3.38 database using the Mascot search engine. The parameters for Mascot searches were as follows:
Type of search : MS/MS Ion Search
Enzyme: Trypsin
Fixed modifications : Carbamidomethyl (C)
Variable modifications : Oxidation (M, Acetyl (N-term, Pyro-glu (N-term Q)
Mass values: Monoisotopic
Protein Mass: Unrestricted
Peptide Mass Tolerance : ± 10 ppm (Orbitrap); ± 2.0 Da (LTQ)
Fragment Mass Tolerance: ± 0.5 Da (LTQ)
Max Missed Cleavages : 1

Mascot output files were parsed into the Scaffold program (www.proteomesoftware.com) for collation into non-redundant lists per lane and filtering to assess false discovery rates and allow only correct protein identifications. Spectral counts per protein were output. These spectral counts constitute a semi-quantitative measure of abundance across samples. Spectral count reflects the number of matched peptides and the number of times those peptides were observed

### Result

A total of 435 proteins were identified. Using the boxplot and student *t*-test methods, 42 proteins were identified having a p-value < 0.05. Further statistical analysis was performed using exploratory data analysis and principal component analysis with S-plus statistical software to examine the differentiation between diseased samples and normal samples of the 42 markers. Results from the principal component analysis along with biological/clinical relevance of the markers led to the set of 20 biomarkers listed in Table 1. MRM-MS assay was set-up to validate those 20 proteins as described in Example 2. Based on the quality of the fragmentation data of the product ion spectra, 10 proteins were chosen

### Example 2: Immunodetection of the biomarkers

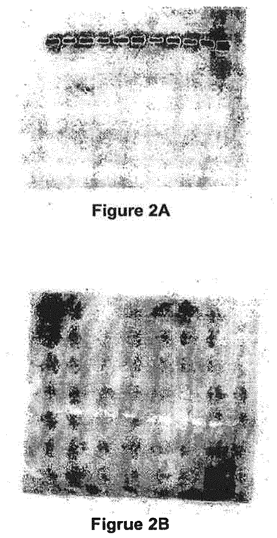

To validate the expression of the biomarkers listed in Table 1, western immunoblotting was performed. Antibodies for ORM1, GSN, SAA, PROZ, PON3, MCAM1, PZP, and FN1 were obtained from Santa Cruz Biotechnology Inc. A 1µl aliquot of each of 11 serum samples from the discovery phase was solublized in SDS-PAGE loading buffer and loaded onto a 4-12% Bis-Tris Novex gel (Invitrogen). The gel was electrophoresed at 120 volts for 60 minutes and the serum proteins separated in the SDS-PAGE gel were transferred to a nitrocellulose membrane using Bio-Rad semi-dry electroblotting unit for 90 min. The blot was blocked with Starting Block™ Blocking Buffer (Pierce) and incubated overnight at 4°C with primary antibody followed by three 10-minute washes with TBS containing 0.05% Tween 20 (TBS-T). The blot was then incubated with a Horseradish Peroxidase ("HRP") conjugated secondary antibody for one hour at room temperature and then washed four times in TBS-T for 15 minutes each time. Signal detection was achieved using SuperSignal Substrate (Pierce) and the blots were imaged using the Kodak 2000 Image Station. Figures 2A and 2B show western blots for colorectal cancer and normal serum samples probed with 1µg/ml rabbit polyclonal anti human fibronectin antibodies and 1µg/ml of rabbit IgG1 isotype antibodies as a negative control. Figure 2A shows a western blot for colorectal cancer and normal serum samples probed with 1µg/ml rabbit polyclonal anti human fibronectin antibodies, and Figure 2B shows a western blot for colorectal cancer and normal serum samples probed with rabbit IgG1 isotype antibodies as a negative control. From left to right in both Figure 2A and Figure 2B: lane 1: stage I colon cancer sample, lane 2: normal, age and gender matched with colon cancer samples in lane 1 and lane 3; lane 3: stage IIA colon cancer sample; lane 4: normal, age and gender matched with lane 5; lane 5: stage IIIA colon cancer sample; lane 6: stage IIIB colon cancer sample; lane 7: normal, age and gender matched with colon cancer sample in lane 6; lane 8:stage IV colon cancer sample; lane 9: normal, age and gender matched with colon cancer sample in lane 8; lane 10: stage I colon cancer sample; land 11: normal, age and gender matched with colon cancer sample in lane 10. Each lane was loaded with 1 µl of serum sample.

### Example 3: Development and validation of an MRM-MS assay MRM-MS peptide panel selection

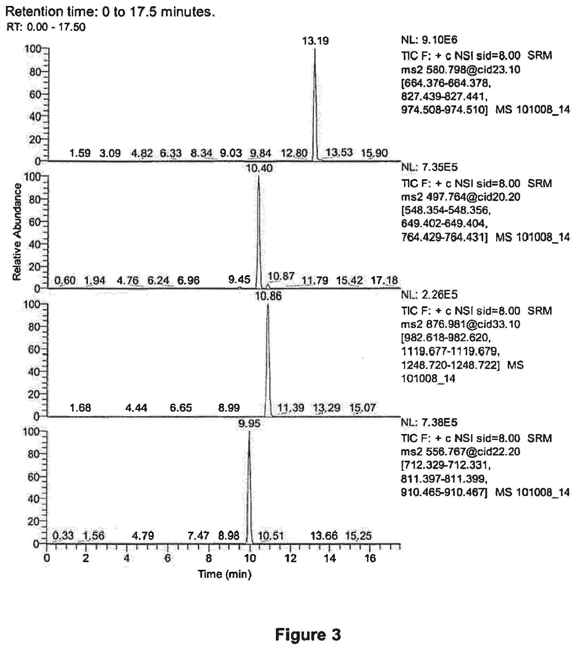

Peptides of the biomarkers listed in Table 1 were selected from discovery data for MRM-MS assay development. All proteins were tested individually in panels with peptides amenable to MRM-MS-assay analysis on pooled disease and control samples. For peptides that worked the best, the method was reduced to a scan for the two most abundant product ions, and the method was run again against both the disease and control pool again. The 10 proteins were chosen as an MRM-MS panel for multiplexing with the best peptide(s) selection per protein. Figure 3 shows representative selected-reaction-monitoring mass spectrometry ("SRM-MS") chromatograms for α-1-acid glycoprotein 1 ("ORM 1") working peptides.

### 10-plex relative protein MRM-MS assay

A 10-plex relative protein assay was developed for these 10 biomarkers. A summary of the sequences of the transition peptides used for MRM-MS assay for the 10 biomarkers is listed in Table 3. The performance of the assay was determined by analyzing normal samples in Example 1 in triplicate from the sample preparation through mass spectrometry to evaluate the reproducibility of the assay.

### Depletion

(1) 15µL of serum was depleted using the MARS7 spin column (Agilent) according to the manufacturers protocol; (2) samples were buffer exchanged using a 5KDa MWCO spin filter into 25mM ammonium bicarbonate; (3) a Bradford protein quantitation assay was performed.

### Solution digestion

Samples were subjected to proteolytic digestion as follows: (1) reducing with DTT at 60°C and allowed to cool to room temperature; (2) alkylating with iodoacetamide and incubated at 37°C for 18h in the presence of trypsin; and (3) adding formic acid to stop the reaction, followed by direct analysis of the supernatant.

### LC/MRM-MS

Peptides were separated using a 15cm x 100µm ID column packed with a 4µm C12 resin (Jupiter Proteo, Phenomenex) under gradient conditions at a constant flow rate of 800nL/min. The gradient is outlined in Table 2. The composition of solvent A was water containing 0.1% formic acid and 0.1% acetonitrile and the composition of solvent B was acetonitrile containing 0.1% formic acid. Samples were loaded onto the column using a trapping strategy. An injection volume of 30µL was used and the experiment was optimized so that 500ng of peptide was loaded on the column per sample. The total runtime, injection to injection, was 20 minutes.

**Table 2: Outline of LC gradient for Solvent A and Solvent B**

| **Time (min)** | **% Solvent A** | **% Solvent B** |
|---|---|---|
| 0.00 | 99 | 1 |
| 10.00 | 75 | 25 |
| 12.00 | 50 | 50 |
| 13.00 | 5 | 95 |
| 14.00 | 5 | 95 |
| 14.10 | 99 | 1 |
| 17.00 | 99 | 1 |

A ThermoFinnigan tandem quadrupole ("TSQ Ultra") mass spectrometer was used for peptide detection in SRM mode. Mass spectrometer settings included a spray voltage of 2.2 kV and capillary temperature of 250°C. A 0.2 FWHM resolution in Q1 (hSRM) and 0.7 FWHM resolution in Q3 were employed. Argon was used as a collision gas at a pressure of 1.5 mTorr. The dwell time for each SRM transition was 10ms. All MRM-MS experiments were conducted in triplicate and the data were processed using the LCQuan software package (ThermoFinnigan).

### Result

A summary of the percent analytical relative standard deviation ("%RSD") and technical %RSD of one transition peptide for all 10 proteins is given in Table 4. Low analytical %RSD (<10%) and technical %RSD (<20%) for transition peptides were achieved, except for the C2 protein. Analytical %RSD is the percent relative standard deviation from a single sample processed via 3 injections; technical %RSD is the percent relative standard deviation from one sample processed 3 times (3 depletion, 3 digestions) with one injection for each 3 separated run.

**Table 3: Summary of transition peptides for 10 proteins**

| **Proteins** | **Transition peptides sequences** | | |
|---|---|---|---|
| | Peptide 1 | Peptide 2 | Peptide 3 |
| ORM1 | SEQ ID NO 1: WFYIASAFR | SEQ ID NO 2: TEDTIFLR | SEQ ID NO 3: SDWYTDWK |
| GSN | SEQ ID NO 4: IFVWK | SEQ ID NO 5: QTQVSVLPEGGETP LFK | SEQ ID NO 6: AGALNSNDAFVL K |
| C9 | SEQ ID NO 7: YAFELK | SEQ ID NO 8: LSPIYNLVPVK | SEQ ID NO 9: AIEDYIEFSVR |
| FN1 | SEQ ID NO 10: WLPSSSPVTGYR | SEQ ID NO 11: IYLYTLNDNAR | SEQ ID NO 12: SYTITGLQPGTD YK |
| SERPIN A3 | SEQ ID NO 13: EQLSLLDR | SEQ ID NO 14: EIGELYLPK | SEQ ID NO 15: ITLLSALVETR |
| PZP | SEQ ID NO 16: ATVLNYLPK | SEQ ID NO 17: AVGYLITGYQR | |
| C2 | SEQ ID NO 18: HAFILQDTK | SEQ ID NO 19: AVISPGFDVFAK | |
| PROZ | SEQ ID NO 20: GLLSGWAR | | |
| PRG4 | SEQ ID NO 21: AIGPSQTHTIR | | |
| SAA2 | SEQ ID NO 22: SFFSFLGEAFDG AR | | |

**Table 4: Summary of analytical and technical %RSD of one transition peptide for 10 proteins**

| **Proteins** | **Peptide 1** | |
|---|---|---|
| | Analytical %RSD | Technical %RSD |
| ORM1 | 3% | 8% |
| GSN | 9% | 15% |
| C9 | 7% | 12% |
| FN1 | 1% | 17% |
| SERPINA3 | 6% | 11 % |
| PZP | 5% | 18% |
| C2 | 16% | 25% |
| PROZ | 8% | 12% |
| PRG4 | 3% | 11% |
| SAA2 | 10% | 24% |

The selected tryptic peptides can be quantified against a stable isotope-labeled peptide as an internal standard to provide a measure of the concentration of the protein, or can be quantified relatively by comparing the expression level against a normal control

### Example 4: Validation of 10 biomarkers with the first expanded 33 serum sample set

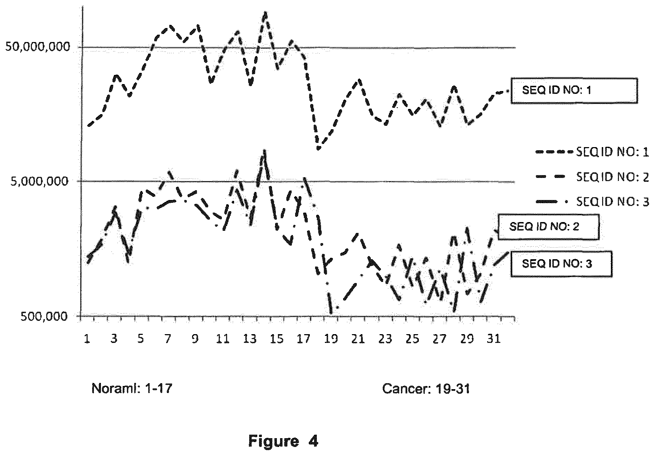

The relative level of 10 protein biomarkers of colon cancer was monitored in 33 patient serum samples as well as in the 13 samples used for biomarker discovery. This larger sample set included 18 age-and-gender-matched normal serum samples and 15 colon cancer serum samples: four Stage I, five Stage II, eight Stage III and one Stage IV. The 33 serum samples were collected from the same institute using the same collection protocol. Thirteen of the fourteen original samples from Example 1 were also tested in the assay. All samples were processed with equivalent amounts of protein. Data from two analytical replicates of each sample were collected and analyzed. The data summary below reports the ratio of the average data for each protein across the samples from a particular stage of disease relative to the average for the normal group. Figure 4 shows representative MRM-MS peptide trend lines of three ORM1 working peptides for 33 serum samples. Sample ID numbers from 38715 to 38732 are normal subjects; sample ID numbers from 38733 to 38750 are from subjects with colon cancer.

### Sample preparation steps

The sample order was randomized so that samples from the same group were not processed in sequence. Samples were prepared following the steps: (1) depletion; (2) solution digestion, as described in Example 3, except that the samples were placed in a 96 well plate and digested with trypsin overnight.

### LC/MRM-MS

An internal standard was added to each sample. Samples were then tested following the same LC/MRM-MS condition as described in Example 3.

### Result

| sample | Stage 1: Normal | p-val | Stage 2: Normal | p-val | Stage 3: Normal | p-val | Stage 4: Normal | p-val |
|---|---|---|---|---|---|---|---|---|
| SERPINA3 | 2.04 | 0.008151 | 3.74 | 0.000243 | 4.60 | 0.011396 | 1.79 | NA |
| FN1 | 0.71 | 0.347941 | 0.59 | 0.125433 | 0.79 | 0.460251 | 0.45 | NA |
| SAA2 | 6.43 | 0.006389 | 19.88 | 0.001009 | 14.24 | 0.012542 | 31.60 | NA |
| PROZ | 0.80 | 0.331598 | 0.69 | 0.186481 | 1.08 | 0.701716 | 0.61 | NA |
| PZP | 0.91 | 0.835463 | 1.73 | 0.088589 | 2.20 | 0.030897 | 0.85 | NA |
| C9 | 1.61 | 0.026879 | 2.73 | 0.000015 | 2.91 | 0.000366 | 2.55 | NA |
| PRG4 | 1.04 | 0.919156 | 1.65 | 0.182810 | 1.78 | 0.040367 | 1.34 | NA |
| C2 | 1.12 | 0.805110 | 2.16 | 0.006040 | 1.55 | 0.130820 | 0.87 | NA |
| GSN | 0.55 | 0.022829 | 0.30 | 0.000224 | 0.83 | 0.447100 | 0.28 | NA |
| ORM1 | 1.41 | 0.095748 | 3.24 | 0.000000 | 3.24 | 0.000054 | 2.37 | NA |

All ten of the biomarkers showed differential expression. Seven of the ten biomarkers discovered were confirmed in this set of serum samples as differentially expressed between the cancer and normal groups with a p-value less than 0.05 in one or more stages of colorectal cancer.

### Statistical analysis

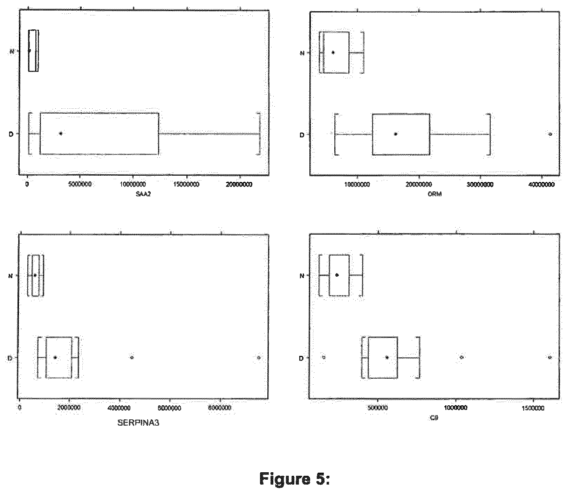

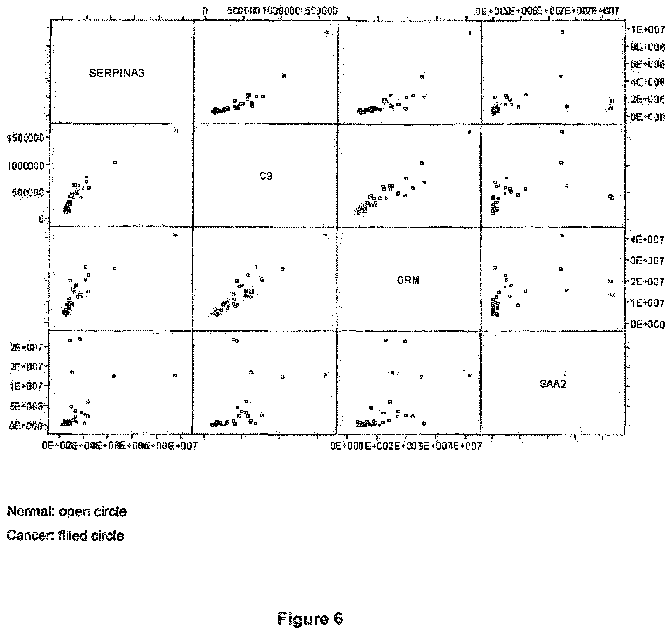

Using S-plus statistical software, exploratory data analysis, multivariate analysis, and discriminant analysis were performed. Figure 5 shows boxplots of cancer vs. normal for serum values for amyloid A protein ("SAA2"), ORM1, plasma serine protease inhibitor ("SERPINA3"), and C9 complement component ("C9"). In Figure 5, the x-axis indicates the spectral counts and the y-axis indicates the sample category: Cancer and Normal. Figure 6 shows a matrix plot for SERPINA3, ORM1, SAA2, and C9. The labels along the x and y axes indicates the spectral counts. The circle indicates the normal controls, and the triangles are the cancer group. A separation between cancer and normal control is observed, which suggests that the difference between the cancer and normal states may be related to a combination of these variables (*i.e.,* biomarkers).

Multivariate analysis and discriminant analysis was carried out for different combinations of multiple biomarkers using 4 markers as classifiers of diseased state vs. normal state: C9, ORM1, SAA2, and SERPINA3. 16 of the 17 cancer samples were classified correctly as cancer, and 15 of the 15 normal samples were classified as normal. The plug-in classification table, using 4 markers shown below, is the output result from S-plus software:

| | Diseased | Normal | Error | Posterior Error |
|---|---|---|---|---|
| Diseased | 16 | 1 | 0.0588235 | 0.00695156 |
| Normal | 0 | 15 | 0.0000000 | 0.0653836 |
| Overall | | | 0.0588235 | 0.0062816 |

Discriminant analysis used 7 markers as classifiers of diseased state vs. normal state: C9, ORM1, SAA2, SERPINA3, PZP, PRG4, and PROZ. 16 of the 17 cancer samples were classified correctly as cancer, and 15 of the 15 normal samples were classified as normal. Output result from S-plus software is shown below in classification table using 7 markers:

| | Diseased | Normal | Error | Posterior Error |
|---|---|---|---|---|
| Diseased | 16 | 1 | 0.0588235 | 0.0588235 |
| Normal | 0 | 15 | 0.0000000 | 0.0663935 |
| Overall | | | 0.0588235 | 0.0001280 |

Discriminant analysis using 8 markers: C9, ORM1, SAA2, SERPINA3, PZP, PRG4, PROZ, and GSN as classifiers of diseased state vs. normal state with heteroscedastic covariance structure. 17 of the 17 cancer samples were classified correctly as cancer, and 15 of the 15 normal samples were classified as normal. Output result from S-plus software as plug-in classification table is shown below:

| | Diseased | Normal | Error | Posterior Error |
|---|---|---|---|---|
| Diseased | 17 | 0 | 0 | 0.0e+000 |
| Normal | 0 | 15 | 0 | 9.8e-006 |
| Overall | | | 0 | 4.6e-006 |

The use of multiple biomarkers increases the predictive value of the test and provides great clinical utility in diagnosis, patient stratification, and patient monitoring.

### Example 5: Validation of 10 biomarkers in plasma sample set

The 10-plex relative protein MRM-MS assay was tested in plasma samples. All samples were collected before treatment and before surgery. 5 plasma colorectal cancer samples, including one Stage I, two Stage II, two Stage III, and one pooled normal plasma sample were tested along with a normal serum and a colorectal cancer serum samples. Samples were prepared and processed to LC/MRM-MS as described in Example 3.

### LC/MRM-MS

An internal standard was added to each sample. Samples were then tested following the same LC/MRM-MS condition, as described in Example 3.

### Result:

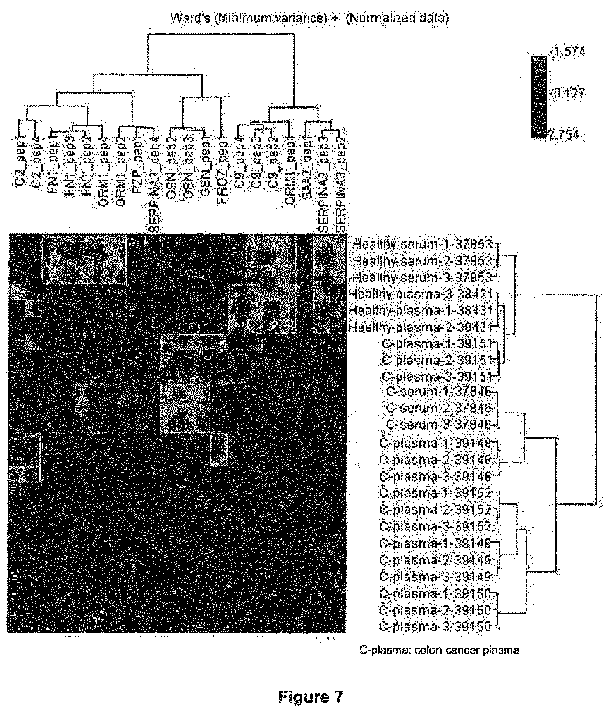

Other than PROG4, all biomarkers were detected. All of the transition peptides of the nine biomarkers discovered were confirmed, except PZP_pep2 (SEQ ID NO: 17) in this set of plasma samples as differential expressed between the cancer and normal groups. The degree of change and the p-value for each transition peptide of the 9 biomarkers are listed in Table 5. All the transition peptides of the nine biomarkers showed a p-value less than 0.05, except C2_pep1(SEQ ID NO: 18), SAA2 (SEQ ID NO: 22), and PROZ_pep1(SEQ ID NO 20). Figure 7 shows a hierarchical clustering analysis of plasma and serum samples. Figure 7 is the Hierarchical Clustering Analysis of plasma and serum samples, where C-plasma denoted Colon cancer plasma sample.

**Table 5: Fold change and p-value of the transition peptides for the 9 biomarkers detected**

| **Proteins peptids** | **Transition peptides sequences** | **Fold change cancer:normal** | **P-value** |
|---|---|---|---|
| C2_pep1 | SEQ ID NO 18: HAFILQDTK | 6.95 | 0.217062141 |
| C2_pep2 | SEQ ID NO 19: AVISPGFDVFAK | 2.78 | 0.002870429 |
| C9_pep1 | SEQ ID NO 7: YAFELK | 2.83 | 1.30572E-05 |
| C9_pep2 | SEQ ID NO 8: LSPIYNLVPVK | 4.49 | 1.05609E-06 |
| C9_pep3 | SEQ ID NO 9: AIEDYIEFSVR | 4.50 | 0.000124535 |
| FN1_pep1 | SEQ ID NO 10: WLPSSSPVTGYR | 3.34 | 0.000265144 |
| FN1_pep2 | SEQ ID NO 11: IYLYTLNDNAR | 3.22 | 0.000460216 |
| FN1_pep3 | SEQ ID NO 12: SYTITGLQPGTDYK | 3.39 | 0.000148397 |
| GSN_pep1 | SEQ ID NO 4: IFVWK | 1.71 | 0.021196134 |
| GSN_pep2 | SEQ ID NO 5: QTQVSVLPEGGGETPLF K | 2.46 | 0.00175376 |
| GSN_pep3 | SEQ ID NO 6: AGALNSNDAFVLK | 1.63 | 0.041756746 |
| ORM1_pep1 | SEQ ID NO 1: WFYIASAFR | 5.85 | 2.14067E-05 |
| ORM1_pep2 | SEQ ID NO 2: TEDTIFLR | 4.13 | 0.000362804 |
| ORM1_pep3 | SEQ ID NO 3: SDWYTDWK | 3.31 | 9.58596E-06 |
| SAA2_pep1 | SEQ ID NO 22: SFFSFLGEAFDGAR | 1750.2 | 0.258875784 |
| PZP_pep1 | SEQ ID NO 16: ATVLNYLPK | 2.09 | 0.090043107 |
| PROZ_pep1 | SEQ ID NO 20: GLLSGWAR | 7.37 | 0.346022569 |
| SERPINA3_pep 1 | SEQ ID NO 13: EQLSLLDR | 3.72 | 7.20014E-05 |
| SERPINA3_pep 2 | SEQ ID NO 14: EIGELYLPK | 4.87 | 0.000202835 |
| SERPINA3_pep 3 | SEQ ID NO 15: ITLLSALVETR | 4.27 | 0.001260361 |

### Example 6: Validation of 10 biomarkers with 2^{nd} expended 48 serum samples

The relative levels of the 10 protein biomarkers of colon cancer was further confirmed and validated by obtaining relative quantitation data from 48 serum samples, including samples from healthy individuals and patients with colon cancer. The 48 serum samples were collected from the same institution as the 33 serum samples in Example 4. Of the 48 samples, 24 were from healthy individuals confirmed by negative colonoscopy and 24 from colorectal cancer patients with different stages, including one from Stage I, twelve from Stage II, six from Stage III, and five from Stage IV.

### Sample preparation steps

The sample order was randomized so that samples from the same group were not processed in sequence. Each sample was processed in analytical triplicate (same processed sample on Mass spectrometry three times). Samples were prepared following the steps: (1) depletion; and (2) solution digestion, as described in Example 3, except that the samples were placed in a 96-well plate and digested with trypsin overnight.

### LC/MRM-MS

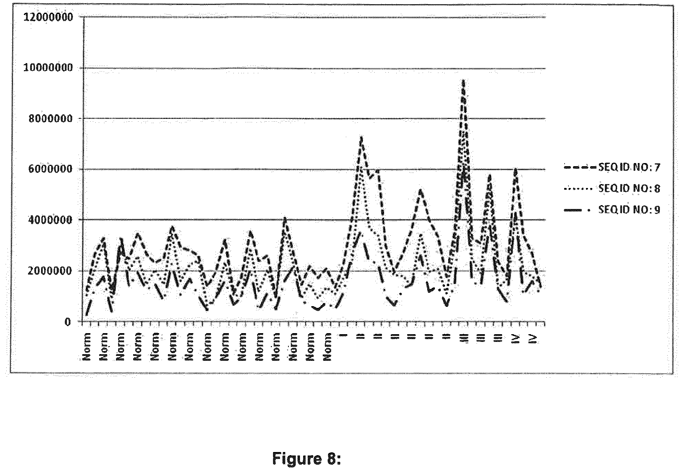

An internal standard was added to each sample. Samples were then tested following the same LC/MRM-MS condition, as described in Example 3. Figure 8 shows an MRM-MS C9 trend line for three transition peptides of the C9 protein.

### Result

Eight of the 10 proteins were detected in these samples. PRG4 and SAA2 were not detected. Four of the proteins (10 peptides) had *p* values less than 0.05 comparing the healthy group to the group with colon cancer. The 4 proteins are: C9, FN1, GSN and SERPINA3. Table 6 provides the *p* values (t-Test) for the different groups (all cancer stages, Stage II, Stage III, and Stage IV compared to the healthy group). Because only one Stage I sample was included in the testing, there is no p value for that sample.

**Table 6: Results at the protein level: p-values of 8 proteins detected from for each group compared to control group**

| | C2 | C9 | FN1 | GSN | ORM1 | PROZ | PZP | SERPIN A3 |
|---|---|---|---|---|---|---|---|---|
| Stage II | 0.797 | 0.054 | 0.001 | 0.264 | 0.682 | 0.241 | 0.657 | 0.121 |
| Stage III | 0.455 | 0.006 | 0.004 | 0.029 | 0.242 | 0.757 | 0.443 | 0.041 |
| Stage IV | 0.694 | 0.409 | 0.529 | 0.170 | 0.283 | 0.279 | 0.556 | 0.006 |
| All cancer | 0.825 | 0.020 | 0.001 | 0.022 | 0.493 | 0.891 | 0.526 | 0.016 |

**Table 7: Results at peptides level: fold change and p-value of the transition peptides for the 8 biomarkers detected**

| **Proteins peptids** | **Transition peptides sequences** | **Fold change cancer:normal** | **P-value** |
|---|---|---|---|
| C2_pep1 | SEQ ID NO 18: HAFILQDTK | 1.06 | 0.54551 |
| C2_pep2 | SEQ ID NO 19: AVISPGFDVFAK | 1.07 | 0.489746 |
| C9_pep1 | SEQ ID NO 7: YAFELK | 1.65 | 0.000325 |
| C9_pep2 | SEQ ID NO 8: LSPIYNLVPVK | 1.52 | 0.007926 |
| C9_pep3 | SEQ ID NO 9: AIEDYIEFSVR | 1.65 | 0.010036 |
| FN1_pep1 | SEQ ID NO 10: WLPSSSPVTGYR | 0.72 | 0.008734 |
| FN1_pep2 | SEQ ID NO 11: IYLYTLNDNAR | 0.67 | 0.000948 |
| GSN_pep1 | SEQ ID NO 4: IFVWK | 0.80 | 0.001601 |
| GSN_pep2 | SEQ ID NO 5: QTQVSVLPEGGETPLF K | 0.80 | 0.140187 |
| GSN_pep3 | SEQ ID NO 6: AGALNSNDAFVLK | 0.81 | 0.009414 |
| ORM1_pep1 | SEQ ID NO 1: WFYIASAFR | 1.31 | 0.190992 |
| ORM1_pep2 | SEQ ID NO 2: TEDTIFLR | 1.10 | 0.493723 |
| ORM1_pep3 | SEQ ID NO 3: SDWYTDWK | 1.22 | 0.222723 |
| PROZ_pep1 | SEQ ID NO 20: GLLSGWAR | 1.00 | 0.982778 |
| PZP_pep1 | SEQ ID NO 16: ATVLNYLPK | 1.10 | 0.212562 |
| PZP_pep2 | SEQ ID NO 17: AVGYLITGYQR | 1.16 | 0.674164 |
| SERPINA3_pep 1 | SEQ ID NO 13: EQLSLLDR | 1.60 | 0.002006 |
| SERPINA3_pep 2 | SEQ ID NO 14: EIGELYLPK | 1.50 | 0.006981 |
| SERPINA3_pep 3 | SEQ ID NO 15: ITLLSALVETR | 1.58 | 0.005854 |

### Statistical Analysis

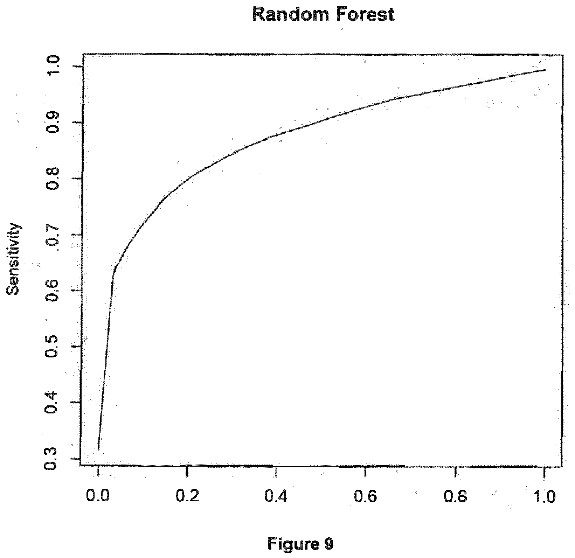

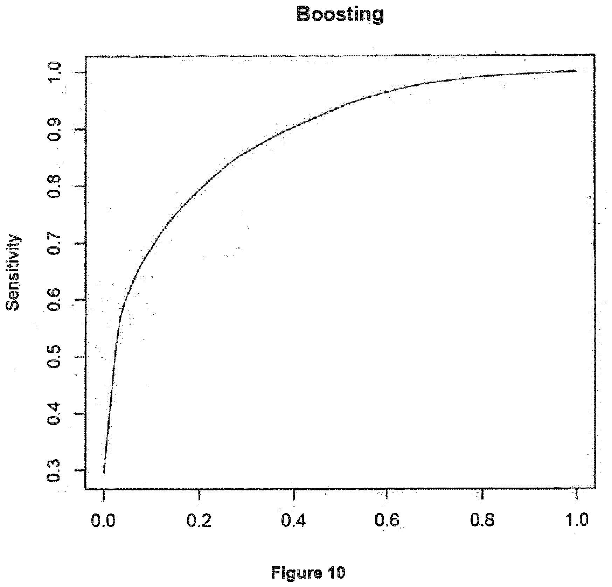

A total of 17 peptides from Table 5, with C2_pep1(SEQ ID NO 18),PROZ_pep1(SEQ ID NO 20) and PZP_pep2(SEQ ID NO 17) excluded, were used for statistical analysis. Two classification methods, Random Forest and Boosting, were used to construct Receiver operating Characteristic ("ROC") curves to assess the diagnostic accuracy of the biomarkers in distinguishing patients with colon cancer from control subjects. The analysis was performed as follows: (1) 48 serum samples were randomly split into a training set of 32 samples and a test set of 16 samples; (2) with a given random split, the training set data was fitted into Random Forest and Boosting models. They were evaluated on the test data set, and variable importance and area under ROC curves were recorded; and (3) steps (1) and (2) were repeated 100 times based on 100 random splits. The final results were averaged over the 100 random splits. Figure 9 shows a Receiver Operating Characteristic ("ROC") curve for a 48-serum-sample set using the random-forest model. Figure 9 is the Receiver Operating Characteristic ("ROC") curve for 48 serum samples set using Random Forest model. The area under curve ("AUC") was 0.868 with an 8.7% standard deviation. Figure 10 shows an ROC curve for 48 serum samples using the boosting method. The area under curve("AUC") is 0.901 and a standard deviation of 5.4% was obtained. Based on both methods, a sensitivity of 80% and specificity of 80% were obtained.

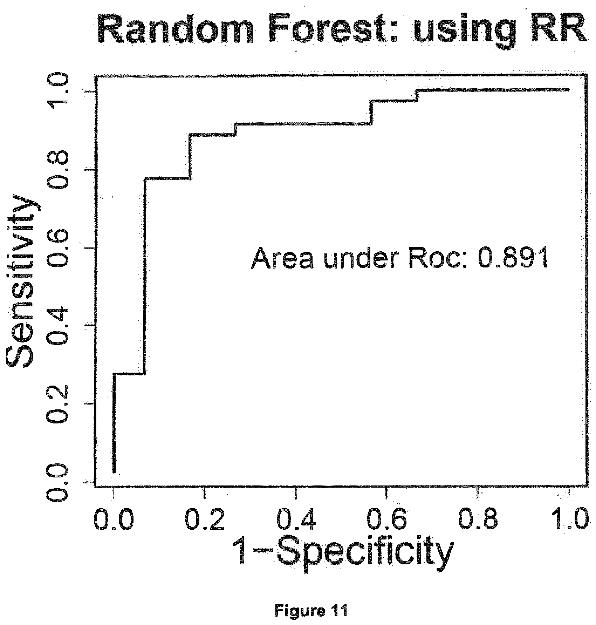

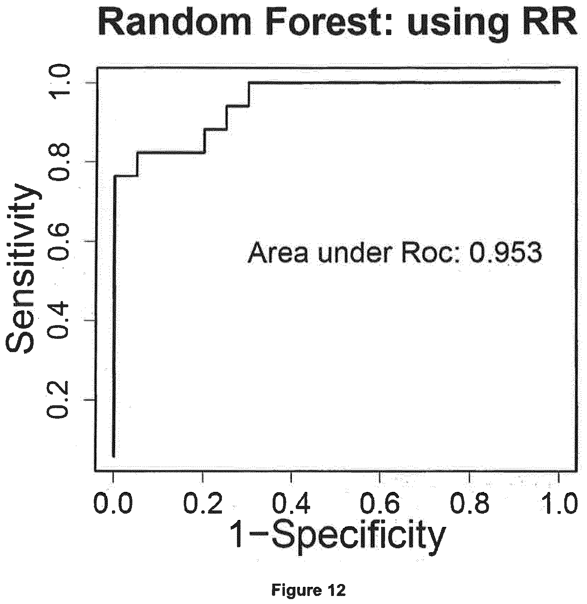

Figure 11 shows an ROC curve for a 33-serum-sample set constructed by the random-forest model. Further statistical analysis based on the Random Forest model was performed using 48 samples set as training set to test the 33 serum samples set in Example 4. The ROC curve is shown in Figure 11 with an AUC of 0.891. A specificity of 90% and sensitivity of 85% was drawn based on the ROC curve. Statistical analysis using Random Forest method was also carried out for a set of 33 serum samples and for the set of 13 serum samples in Example 4. Figure 12 shows an ROC curve for a 13-serum-sample set constructed by the random-forest model. The ROC curve is shown in Figure 12 using a set of 33 serum samples as a training set to test the 13 serum samples set. An AUC of 0.953 was obtained and giving a sensitivity of around 83% and specificity around 95%.

### Example 7: Absolute quantitative MRM-MS assay development

An absolute quantitative MRM-MS assay using stable isotope dilution mass spectrometry was further developed. In this study, quantification of proteins is accomplished by selecting "signature" peptides derived by trypsin digestion of the target protein released during sample digestion. These signature peptides, unique in sequence (i.e. not present in other proteins in the genome), are used as quantitative, stoichiometric surrogates of the protein itself. When a synthetic, stable isotope-labeled version is used as an internal standard, protein concentration can be measured by comparing the signals from the exogenous labeled and endogenous unlabeled species. To insure optimal performance of peptide standards used for quantitation, alternative peptides other than the transition peptides listed in Table 3 for each protein in Table 1 were also evaluated.

### Peptide selection and detection:

Peptides candidates for each protein were generated based on their presence in the serum spectral library, which is a collation of all peptides observed during discovery experiments described in Example 1, and their physical properties, such as size, amino acid composition.

The six control and six disease samples were used to generate two pools: pool-control and pool-disease. A method for each protein including all the peptides candidates was used to run both samples. The mass chromatograms were inspected visually using the Skyline program. Peptides were eliminated when they fell into one or more of these criteria: (1) no peak in either sample; (2) peak detected, but product ion ratio is not similar; (3) multiple peaks detected which could cause potential interference. A variance test was also performed where a pooled sample (3 controls and 3 diseases) was prepared in triplicate (varA, varB, and varC). Each of these three samples was then analyzed in triplicate. Samples were processed following the steps: (1) depletion; and (2) solution digestion, as described in Example 3, except that 10 µl of serum was depleted instead of 15 µl of serum. Samples were then tested following the same LC/MRM-MS condition as described in Example 3. Peptides with analytical and technical variance greater than 20% were eliminated.

### Multiplex assay testing:

Based on the peptides selected, a multiplex assay was constructed. To further evaluate the robustness of the multiplex assay with the selected signature peptides, a variance test and a pilot test were carried out. Table 8 is a list of selected peptides for the multiplex assay and pilot testing.

**Table 8: List of selected signature peptides for multiplex assay**

| **Proteins** | **Selected signature peptides sequences** | | |
|---|---|---|---|
| ORM1 | SEQ ID NO 1: WFYIASAFR | SEQ ID NO 2: TEDTIFLR | SEQ ID NO 3: SDWYTDWK |
| | SEQ ID NO 23 YVGGQEHFAHLLIL R | | |
| GSN | SEQ ID NO 4: IFVWK | SEQ ID NO 5: QTQVSVLPEGGETPL FK | SEQ ID NO 6: AGALNSNDAFVL K |
| | SEQ ID NO 24 HVVPNEVVVQR | SEQ ID NO 25 SEDCFILDHGK | |
| C9 | SEQ ID NO 8: LSPIYNLVPVK | SEQ ID NO 9: AIEDYIEFSVR | SEQ ID NO 26 SIEVFGQFNGK |
| | SEQ ID NO 27 TSNFNAAISLK | | |
| FN1 | SEQ ID NO 10: WLPSSSPVTGYR | SEQ ID NO 11: IYLYTLNDNAR | SEQ ID NO 12: SYTITGLQPGTD YK |
| | SEQ ID NO 28 VTWAPPPSIDLTNF LVR | | |
| SERPINA3 | SEQ ID NO 13: EQLSLLDR | SEQ ID NO 15: ITLLSALVETR | SEQ ID NO 29 ADLSGITGAR |
| | SEQ ID NO 30: AVLDVFEEGTEASA ATAVK | | |
| PZP | SEQ ID NO 16: ATVLNYLPK | SEQ ID NO 17: AVGYLITGYQR | SEQ ID NO 31: SLFTDLVAEK |
| | SEQ ID NO 32: NQGNTWLTAFVLK | SEQ ID NO 33: SSGSLLNNAIK | |
| C2 | SEQ ID NO 18: HAFILQDTK | SEQ ID NO 19: AVISPGFDVFAK | SEQ ID NO 34: ECQGNGVWSG TEPICR |
| | SEQ ID NO 35 EILNINQK | SEQ ID NO 36: DFHINLFR | |
| PROZ | SEQ ID NO 20: GLLSGWAR | SEQ ID NO 37: APDLQDLPWQVK | SEQ ID NO 38: ENFVLTTAK |
| | SEQ ID NO 39: YSLWFK | | |
| PRG4 | SEQ ID NO 40: ITEVWGIPSPIDTVF TR | SEQ ID NO 41: GFGGLTGQIVAALST AK | SEQ ID NO 42: IQYSPAR |
| | SEQ ID NO 43: DQYYNIDVPSR | SEQ ID NO 44: CFESFER | |
| SAA2 | SEQ ID NO 22: SFFSFLGEAFDGAR | SEQ ID NO 45: GPGGVWAAEAISDAR | SEQ ID NO 46: FFGHGAEDSLA DQAANEWGR |
| CFHR2 | SEQ ID NO 47: ITCAEEGWSPTPK | SEQ ID NO 48: GWSTPPK | SEQ ID NO 49: TGDIVEFVCK |
| | SEQ ID NO 50: LVYPSCEEK | | |
| LOC653879 | SEQ ID NO 51: IHWESASLLR | SEQ ID NO 52: NTLIIYLDK | SEQ ID NO 53: VYAYYNLEESCT R |
| | SEQ ID NO 54: ACEPGVDYVYK | SEQ ID NO 55: TFISPIK | |
| HABP2 | SEQ ID NO 56: FTCACPDQFK | SEQ ID NO 57: VVLGDQDLK | SEQ ID NO 58: LIANTLCNSR |
| | SEQ ID NO 59: FLNWIK | | |

### Variance test

A pooled sample comprising three controls and three diseases was prepared three times to give the following samples: varA, varB, and varC. Each of these three samples was analyzed in three runs. The three analytical runs were done on three different days. Samples were processed following the steps: (1) depletion; and (2) solution digestion, as described in Example 3, except that 10 µl of serum was depleted instead of 15 µl of serum. An internal standard was added to each sample. Samples were then tested following the same LC/MRM-MS condition as described in Example 3.

### Pilot test

Twelve serum samples, including 6 normal serum samples and 6 colorectal cancer serum samples (2 stage II, 3 stage III and 1 stage IV), were run in triplicate. The disease-to-control ratios and the p-value for 13 proteins in the multiplex assay are listed in Table 9. The disease-to-control ratios and p-values of the selected peptides in the multiplex assay for 13 proteins at the peptide level are listed in Table 10.

**Table 9: Multiplex pilot test result at protein level: the disease-to-control ratio and p-value.**

| Protein | Ratio: Disease/control | p-value |
|---|---|---|
| ORM1 | 3.12 | 0.0008 |
| GSN | 0.34 | 0.0001 |
| C9 | 2.97 | 0.0001 |
| FN1 | 0.33 | 0.0003 |
| SERPINA3 | 2.84 | 0.000007 |
| PZP | 3.05 | 0.1086 |
| C2 | 1.34 | 0.0541 |
| PROZ | 0.72 | 0.1664 |
| PRG4 | 0.81 | 0.2014 |
| SAA2 | 52.60 | 0.0066 |
| CFHR2 | 0.68 | 0.0499 |
| LOC65387 | 0.97 | 0.8451 |
| HABP2 | 1.14 | 0.3621 |

**Table 10: Multiplex pilot test result at peptide level: the disease-to-control ratio and p-value.**

| Protein | Peptide | Ratio: Disease/Normal | p-Value |
|---|---|---|---|
| ORM1 | SEQ ID NO 1: WFYIASAFR | 2.50 | 0.0033156 |
| ORM1 | SEQ ID NO 2: TEDTIFLR | 2.47 | 0.0003729 |
| ORM1 | SEQ ID NO 3: SDWYTDWK | 3.87 | 0.0000128 |
| ORM1 | SEQ ID NO 23 YVGGQEHFAHLLILR | 3.86 | 0.0010666 |
| GSN | SEQ ID NO 4: IFVWK | 0.32 | 0.0000422 |
| GSN | SEQ ID NO 5: QTQVSVLPEGGETPLFK | 0.32 | 0.0000399 |
| GSN | SEQ ID NO 6: AGALNSNDAFVLK | 0.34 | 0.0000089 |
| GSN | SEQ ID NO 24 HVVPNEVVVQR | 0.32 | 0.0000097 |
| GSN | SEQ ID NO 25 SEDCFILDHGK | 0.31 | 0.0000336 |
| C9 | SEQ ID NO 8: LSPIYNLVPVK | 3.08 | 0.0000184 |
| C9 | SEQ ID NO 9: AIEDYIEFSVR | 3.24 | 0.0001148 |
| C9 | SEQ ID NO 26 SIEVFGQFNGK | 2.94 | 0.0000027 |
| C9 | SEQ ID NO 27 TSNFNAAISLK | 2.79 | 0.0000551 |
| FN1 | SEQ ID NO 10: WLPSSSPVTGYR | 0.28 | 0.0000131 |
| FN1 | SEQ ID NO 11: IYLYTLNDNAR | 0.28 | 0.0000457 |
| FN1 | SEQ ID NO 12: SYTITGLQPGTDYK | 0.30 | 0.0000578 |
| FN1 | SEQ ID NO 28 VTWAPPPSIDLTNFLVR | 0.33 | 0.0010085 |
| SERPINA3 | SEQ ID NO 13: EQLSLLDR | 3.24 | 0.0000512 |
| SERPINA3 | SEQ ID NO 15: ITLLSALVETR | 2.94 | 0.0000022 |
| SERPINA3 | SEQ ID NO 29 ADLSGITGAR | 3.10 | 0.0000062 |
| SERPINA3 | SEQ ID NO 30: AVLDVFEEGTEASAATAVK | 2.61 | 0.0000085 |
| PZP | SEQ ID NO 16: ATVLNYLPK | 1.04 | 0.8121794 |
| PZP | SEQ ID NO 17: AVGYLITGYQR | 7.97 | 0.1318286 |
| PZP | SEQ ID NO 31: SLFTDLVAEK | 7.44 | 0.1350599 |
| PZP | SEQ ID NO 32: NQGNTWLTAFVLK | 1.07 | 0.7614844 |
| PZP | SEQ ID NO 33: SSGSLLNNAIK | 1.00 | 0.9896744 |
| C2 | SEQ ID NO 18: HAFILQDTK | 1.40 | 0.0324419 |
| C2 | SEQ ID NO 19: AVISPGFDVFAK | 1.39 | 0.0104918 |
| C2 | SEQ ID NO 34: ECQGNGVWSGTEPICR | 1.23 | 0.1953067 |
| C2 | SEQ ID NO 35: EILNINQK | 1.29 | 0.1055677 |
| C2 | SEQ ID NO 36: DFHINLFR | 1.32 | 0.0238423 |
| PROZ | SEQ ID NO 20: GLLSGWAR | 0.79 | 0.3017053 |
| PROZ | SEQ ID NO 39: YSLWFK | 0.51 | 0.2008887 |
| PRG4 | SEQ ID NO 40: ITEVWGIPSPIDTVFTR | 0.73 | 0.2066627 |
| PRG4 | SEQ ID NO 41: GFGGLTGQIVAALSTAK | 0.75 | 0.1399844 |
| PRG4 | SEQ ID NO 42: IQYSPAR | 0.93 | 0.7253475 |
| PRG4 | SEQ ID NO 43: DQYYNIDVPSR | 0.84 | 0.3454110 |
| PRG4 | SEQ ID NO 44: CFESFER | 0.67 | 0.0228997 |
| SAA2 | SEQ ID NO 22: SFFSFLGEAFDGAR | 41.34 | 0.0017767 |
| SAA2 | SEQ ID NO 45: GPGGVWAAEAISDAR | 102.78 | 0.0095953 |
| SAA2 | SEQ ID NO 46: FFGHGAEDSLADQAANEWGR | 57.81 | 0.0012913 |
| CFHR2 | SEQ ID NO 47: ITCAEEGWSPTPK | 1.76 | 0.0268359 |
| CFHR2 | SEQ ID NO 48: GWSTPPK | 0.28 | 0.0000001 |
| CFHR2 | SEQ ID NO 49: TGDIVEFVCK | 1.30 | 0.3480817 |
| CFHR2 | SEQ ID NO 50: LVYPSCEEK | 1.29 | 0.4772370 |
| LOC653879 | SEQ ID NO 51: IHWESASLLR | 0.82 | 0.4210371 |
| LOC653879 | SEQ ID NO 52: NTLIIYLDK | 0.90 | 0.4952480 |
| LOC653879 | SEQ ID NO 53: VYAYYNLEESCTR | 1.00 | 0.9992529 |
| LOC653879 | SEQ ID NO 54: ACEPGVDYVYK | 0.96 | 0.7893764 |
| LOC653879 | SEQ ID NO 55: TFISPIK | 0.87 | 0.4728866 |
| HABP2 | SEQ ID NO 56: FTCACPDQFK | 1.01 | 0.9363291 |
| HABP2 | SEQ ID NO 57: VVLGDQDLK | 1.10 | 0.5591393 |
| HABP2 | SEQ ID NO 58: LIANTLCNSR | 1.11 | 0.5277824 |
| HABP2 | SEQ ID NO 59: FLNWIK | 1.20 | 0.1554325 |

Among the 13-protein panel, the following 9 proteins have one or more peptides with a *p*-value less than 0.05: ORM1, GSN, C9, FN1, SERPINA3, C2, PRG4, SAA2, and CFHR2. Other than the peptides SEQ ID NO 23, SEQ ID NO 5, SEQ ID NO 25, SEQ ID NO28, SEQ ID NO31, SEQ ID NO 18, SEQ ID NO 34, SEQ ID NO 44, SEQ ID NO 48, and SEQ ID NO 53 in Table 8, 46 signature peptides were chosen from Table 8. The 92 peptides including 46 light peptides and 46 of heavy isotopes labeled peptides were ordered from Thermo-Fisher Scientific. The heavy isotopes are labeled on the C-terminus lysine or arginine.

The invention is further characterized by the following items
1. A method for detecting colorectal cancer in a subject comprising: obtaining a biological sample from a subject; determining an expression level of the one or more biomarkers listed in Table 1 from the biological sample; comparing the expression level of the one or more biomarkers with corresponding expression levels of the one or more biomarkers in a normal control sample; and based on the comparison, determining the likelihood that the subject has colorectal cancer.
2. The method of item 1, wherein the biological sample is selected from the group consisting of whole blood, blood plasma, serum, urine, tissue sample, cell sample, and tumor sample.
3. The method of item 1, wherein the biological sample is serum.
4. The method of item 1, wherein the expression level of one or more biomarkers in the biological sample from the subject shows a difference as compared with the expression level in the normal control sample, and wherein the difference detects the presence of colorectal cancer in the subject.
5. The method of item 4, wherein the difference is increased and is at least 1.05 fold greater in the subject as compared with the normal control sample, and wherein the difference detects the presence of colorectal cancer the subject.
6. The method of item 4, wherein the difference is decreased and is at least 0.9 less than the normal control sample, and wherein the difference detects the presence of colorectal cancer in the subject.
7. The method of item 4, wherein one or more biomarkers is a protein.
8. The method of item 7, wherein one or more protein biomarkers are selected from ORM1, GSN, C2 complement, C9 complement, PZP, CRP, CFHR1, CFHR2, SERPINA3, HABP2, CNDP1 , CFHR2, SAA2, LOC653879 similar to Complement C3, PROZ, PON3, RARRES2, GGH, PRG4, MCAM₁ and FN1.
9. The method of item 7, wherein one or more protein biomarkers are selected from ORM1 , GSN, C9, FN1 ,SERPINA3, PZP, C2, PROZ, PRG, and SAA2.
10. The method of item 4, wherein the difference is determined by mass spectrometry, immunohistochemistry, ELISA, or Western blotting.
11. The method of item 4, wherein the difference is determined by relative quantitative Multiple Reaction Monitoring (MRM-MS)-LC/MS/MS.
12. The method of item 4, wherein the difference is determined by quantitative Multiple Reaction Monitoring (MRM-MS)-LC/MS/MS using stable isotope-labeled peptides corresponding to peptides derived from one or more biomarkers set forth in Tablei.
13. The method of item 1, wherein one or more biomarkers is nucleic acid.
14. The method of item 13, further comprising comparing the presence or absence, or the amount or concentration, of one or more nucleic acid biomarkers in the biological sample with the presence or absence, or the amount or concentration, of one or more nucleic acid biomarkers in the normal control sample.
15. The method of item 14, wherein the detecting of the presence or absence, or the amount or concentration of, one or more nucleic acid biomarkers is carried out by RT-PCR.
16. A kit for detecting colorectal cancer by comparing the presence or absence, or the amount or concentration, of one or more biomarkers listed in table 1 in the biological sample with the presence or absence, or the amount or concentration, of said one or more biomarkers in the normal control sample, comprising antibodies, or antibody fragments, which selectively bind to the biomarkers, and instructions for use.
17. A kit for detecting colorectal cancer by comparing the presence or absence, or the amount or concentration, of one or more nucleic acid biomarkers in the biological sample with the presence or absence, or the amount or concentration, of one or more nucleic acid biomarkers in the normal control sample, comprising an mRNA extracting buffer, at least one reverse transcription enzyme, at least one pair of primers that have the nucleotide sequences encompassed coding for any one or more of the amino acid residues of the protein biomarkers listed in Table 1 , and instructions for use.
18. A kit for detecting colorectal cancer by comparing the amount or concentration, of one or more biomarkers listed in table 1 in the biological sample with the amount or concentration, of one or more biomarkers in the normal control sample, comprising isotopically labeled peptides corresponding to peptides derived from one or more biomarkers in Table 1, an internal standard, a set of calibrators, and instruction for use.
19. The method of any one of the above items, wherein one or more biomarkers further comprises CEA.
20. The method of any one of the above items, wherein one or more biomarkers further comprises CEA and carbohydrate antigen 19-9.

gtccttgcaa aattgtctaa aatgtcagtg tttgggaaat taaatccaat aaaaacattt 2640
tgaagtgtga aaaaaaaaaa aaaaaaaaaa aaaaaaaaaa aaaaaaaaaa aaaaaaaaaa 2700
aaaaaaaaaa aaaaaaaaa 2719pora[?]
[?]agcurymkswbdhvnm cyt[?]RNA[?] [?] [?]€[?] [?]NCBI/NM_000177
2009-10-18[?]1[?]2719 [?] €[?] soma [?]€[?] [?]V3.3[?]C2[?]homo
sapiensÿ:
ggctctctac ctctcgccgc ccctagggag gacaccatgg gcccactgat ggttcttttt 60
tgcctgctgt tcctgtaccc aggtctggca gactcggctc cctcctgccc tcagaacgtg 120
aatatctcgg gtggcacctt caccctcagc catggctggg ctcctgggag ccttctcacc 180
tactcctgcc cccagggcct gtacccatcc ccagcatcac ggctgtgcaa gagcagcgga 240
cagtggcaga ccccaggagc cacccggtct ctgtctaagg cggtctgcaa acctgtgcgc 300
tgtccagccc ctgtctcctt tgagaatggc atttataccc cacggctggg gtcctatccc 360
gtgggtggca atgtgagctt cgagtgtgag gatggcttca tattgcgggg ctcgcctgtg 420
cgtcagtgtc gccccaacgg catgtgggat ggagaaacag ctgtgtgtga taatggggct 480
ggccactgcc ccaacccagg catttcactg ggcgcagtgc ggacaggctt ccgctttggt 540
catggggaca aggtccgcta tcgctgctcc tcgaatcttg tgctcacggg gtcttcggag 600
cgggagtgcc agggcaacgg ggtctggagt ggaacggagc ccatctgccg ccaaccctac 660
tcttatgact tccctgagga cgtggcccct gccctgggca cttccttctc ccacatgctt 720
ggggccacca atcccaccca gaagacaaag gaaagcctgg gccgtaaaat ccaaatccag 780
cgctctggtc atctgaacct ctacctgctc ctggactgtt cgcagagtgt gtcggaaaat 840
gactttctca tcttcaagga gagcgcctcc ctcatggtgg acaggatctt cagctttgag 900
atcaatgtga gcgttgccat tatcaccttt gcctcagagc ccaaagtcct catgtctgtc 960
ctgaacgaca actcccggga tatgactgag gtgatcagca gcctggaaaa tgccaactat 1020
aaagatcatg aaaatggaac tgggactaac acctatgcag ccttaaacag tgtctatctc 1080
atgatgaaca accaaatgcg actcctcggc atggaaacga tggcctggca ggaaatccga 1140
catgccatca tccttctgac agatggaaag tccaatatgg gtggctctcc caagacagct 1200
gttgaccata tcagagagat cctgaacatc aaccagaaga ggaatgacta tctggacatc 1260
tatgccatcg gggtgggcaa gctggatgtg gactggagag aactgaatga gctagggtcc 1320
aagaaggatg gtgagaggca tgccttcatt ctgcaggaca caaaggctct gcaccaggtc 1380
tttgaacata tgctggatgt ctccaagctc acagacacca tctgcggggt ggggaacatg 1440
tcagcaaacg cctctgacca ggagaggaca ccctggcatg tcactattaa gcccaagagc 1500
caagagacct gccgggggc cctcatctcc gaccaatggg tcctgacagc agctcattgc 1560
ttccgcgatg gcaacgacca ctccctgtgg agggtcaatg tgggagaccc caaatcccag 1620
tggggcaaag aattccttat tgagaaggcg gtgatctccc cagggtttga tgtctttgcc 1680
aaaaagaacc agggaatcct ggagttctat ggtgatgaca tagctctgct gaagctggcc 1740
cagaaagtaa agatgtccac ccatgccagg cccatctgcc ttccctgcac gatggaggcc 1800
aatctggctc tgcggagacc tcaaggcagc acctgtaggg accatgagaa tgaactgctg 1860
aacaaacaga gtgttcctgc tcattttgtc gccttgaatg ggagcaaact gaacattaac 1920
cttaagatgg gagtggagtg gacaagctgt gccgaggttg tctcccaaga aaaaaccatg 1980
ttccccaact tgacagatgt cagggaggtg gtgacagacc agttcctatg cagtgggacc 2040
caggaggatg agagtccctg caagggagaa tctgggggag cagttttcct tgagcggaga 2100
ttcaggtttt ttcaggtggg tctggtgagc tggggtcttt acaacccctg ccttggctct 2160
gctgacaaaa actcccgcaa aagggcccct cgtagcaagg tcccgccgcc acgagacttt 2220
cacatcaatc tcttccgcat gcagccctgg ctgaggcagc acctggggga tgtcctgaat 2280
tttttacccc tctagccatg gccactgagc cctctgctgc cctgccagaa tctgccgccc 2340
ctccatcttc tacctctgaa tggccaccct tagaccctgt gatccatcct ctctcctagc 2400
tgagtaaatc cgggtctcta ggatgccaga ggcagcgcac acaagctggg aaatcctcag 2460
ggctcctacc agcaggactg cctcgctgcc ccacctcccg ctccttggcc tgtccccaga 2520
ttccttccct ggttgacttg actcatgctt gtttcacttt cacatggaat ttcccagtta 2580
tgaaattaat aaaaatcaat ggtttccac 2609e le[?]
[?]agcurymkswbdhvnm<tR [?]RNA[?] [?] [?]€[?] [?]NCBI/NM_000063
2009-02-15[?]1[?]2609 [?] €[?] <tR [?]€[?] [?]V3.3[?]C9[?]homo
sapiensÿ%
gcttgttccc tgtcctctgg ccctttgcaa ataaatgcct taccagacct gccctgccac 60
cccactcgca gccacccagc aagagcagca tgtcagcctg ccggagcttt gcagttgcaa 120
tctgcatttt agaaataagc atcctcacag cacagtacac gaccagttat gacccagagc 180
taacagaaag cagtggctct gcatcacaca tagactgcag aatgagcccc tggagtgaat 240
ggtcacaatg cgatccttgt ctcagacaaa tgtttcgttc aagaagcatt gaggtctttg 300
gacaatttaa tgggaaaaga tgcaccgacg ctgtgggaga cagacgacag tgtgtgccca 360
cagagccctg tgaggatgct gaggatgact gcggaaatga ctttcaatgc agtacaggca 420
gatgcataaa gatgcgactt cggtgtaatg gtgacaatga ctgcggagac ttttcagatg 480
aggatgattg tgaaagtgag ccccgtcccc cctgcagaga cagagtggta gaagagtctg 540
agctggcacg aacagcaggc tatgggatca acatttaggg gatggatccc ctaagcacac 600
cttttgacaa tgagttctac aatggactct gtaaccggga tcgggatgga aacactctga 660

catactaccg aagaccttgg aacgtggctt ctttgatcta tgaaaccaaa ggcgagaaaa 720
atttcagaac cgaacattac gaagaacaaa ttgaagcatt taaaagtatc atccaagaga 780
agacatcaaa ttttaatgca gctatatctc taaaatttac acccactgaa acaaataaag 840
ctgaacaatg ttgtgaggaa acagcctcct caatttcttt acatggcaag ggtagttttc 900
ggttttcata ttccaaaaat gaaacttacc aactattttt gtcatattct tcaaagaagg 960
aaaaaatgtt tctgcatgtg aaaggagaaa ttcatctggg aagatttgta atgagaaatc 1020
gcgatgttgt gctcacaaca acttttgtgg atgatataaa agctttgcca actacctatg 1080
aaaagggaga atattttgcc tttttggaaa cctatggaac tcactacagt agctctgggt 1140
ctctaggagg actctatgaa ctaatatatg ttttggataa agcttccatg aagcggaaag 1200
gtgttgaact aaaagacata aagagatgcc ttgggtatca tctggatgta tctctggctt 1260
tctctgaaat ctctgttgga gctgaattta ataaagatga ttgtgtaaag aggggagagg 1320
gtagagctgt aaacatcacc agtgaaaacc tcatagatga tgttgtttca ctcataagag 1380
gtggaaccag aaaatatgca tttgaactga aagaaaagct tctccgagga accgtgattg 1440
atgtgactga ctttgtcaac tgggcctctt ccataaatga tgctcctgtt ctcattagtc 1500
aaaaactgtc tcctatatat aatctggttc cagtgaaaat gaaaaatgca cacctaaaga 1560
aacaaaactt ggaaagagcc attgaagact atatcaatga atttagtgta agaaaatgcc 1620
acacatgcca aaatggaggt acagtgattc taatggatgg aaagtgtttg tgtgcctgcc 1680
cattcaaatt tgagggaatt gcctgtgaaa tcagtaaaca aaaaatttct gaaggattgc 1740
cagccctaga gttccccaat gaaaaataga gctgttggct tctctgagct ccagtggaag 1800
aagaaaacac tagtaccttc agatcctacc cctgaagata atcttagctg ccaagtaaat 1860
agcaacatgc ttcatgaaaa tcctaccaac ctctgaagtc tcttctctct taggtctata 1920
attttttttt aaatttttct tccttaaact cctgtgatgt ttccattttt tgttccctaa 1980
tgagaagtca acagtgaaat acgccagaac tgctttatcc cacggaaaat gccaatctct 2040
tctaaaaaaa aacaaaatta aattaaaaac agaatgttgg tttaaaaaac ttcaaagtaa 2100
ttttcaaacg gctttgtatg gttaacatat tctgccaggt ccatgaccac acgtctgtac 2160
catgcaattt aactcttatt tacattgtta tgtttagttt ggttatttgc ttaggtgtgc 2220
atacattcat tcagcaaatg ctgagcacca gccacgtgca cagcagttgc ttttactagt 2280
cttagctcta cgatttaaat ccatgtgtcc aagggggaaa acatattata tttgtaacca 2340
aaaactacta gtttaccaga ggactgaagg gagataaaga ggagttggtt aatgggtaca 2400
aaaatccagt tagatgaaag gaataatata gatagtgttc agtagcagaa tagaatgaac 2460
ataaactatt agtttaaatt atgtgaaatt ccttctattt gatcatattt tacaagaaaa 2520
aacatcaatt ttatatagtc caacttaata cctagcctta tgagttgtat aaggtaaggt 2580
tacctacctg agaagctgat taacattggt tgtacaatct tattcattag agaacatggt 2640
gcttagggtc tgagaccttt tgaaaggtct gagaactctt taaaaaaagg aaa 2693
ista[?] [?]agcurymkswbdhvnm sal[?]RNA[?] [?] [?]€[?] [?]NCBI/NM_001737
2009-04-26[?]1[?]2693 [?] €[?] <tR [?]€[?] [?]V3.3[?]PZP[?]homo
sapiensÿ)[?]ggacacaacc ctgagattta tccctcacaa tgcggaaaga cagacttctt catttatgtc
60
ttgtgctact tcttatcctg ctttctgcca gtgactcaaa ctctacagaa ccgcagtata 120
tggtgctggt cccctccctg ctccacactg aggcccctaa gaagggctgt gtccttctga 180
gccacctgaa tgagacagtg actgtaagtg cttccttgga gtctggcagg gaaaacagga 240
gcctcttcac tgacctggtg gcggagaagg acttattcca ctgtgtctcc ttcactctcc 300
caaggatctc agcctcttca gaggtggcat tccttagcat ccagataaag gggcctacgc 360
aagatttcag gaagaggaac acagttctgg tactgaacac ccaaagtctg gtctttgtcc 420
agacagacaa acccatgtat aaaccaggac agacagtaag attccgtgtt gtctccgtgg 480
atgaaaattt tcgccctcga aatgaactga ttccactgat ataccttgag aacccaagaa 540
gaaatcgaat tgcacaatgg cagagtctca agctagaagc tggcatcaat cagttgtcct 600
ttcccctctc atcagagccc attcagggct cctacagggt ggtggtacag acagaatcag 660
gtggaaggat acagcacccc ttcaccgtgg aggaatttgt gcttcccaag tttgaggtca 720
aagttcaggt gccaaagata atcagtatca tggatgaaaa agtgaacata acagtctgtg 780
gagaatacac ttatgggaag cctgtcccag gacttgcaac tgtgagcctg tgtagaaaat 840
tatctcgtgt tcttaattgt gacaagcagg aggtctgtga ggaattcagt caacagctta 900
acagcaatgg ctgcatcacc caacaagtac acaccaaaat gctccagatt acaaatacgg 960
gctttgaaat gaagcttaga gtggaagcca ggatcagaga agaggggaca gacctggaag 1020
tcactgcaaa caggatcagt gaaatcacaa acattgtatc caaactcaaa ttcgtgaaag 1080
tggattcaca ctttagacaa ggaatcccct tttttgcaca ggtgcttctg gtggatggaa 1140
aaggtgtgcc catccccaat aaactcttct tcatctctgt gaatgacgcc aattattact 1200
ccaatgcaac caccaatgag cagggtcttg cacagttttc aatcaatact accagtatct 1260
cggttaataa actttttgtc cgggttttca ctgtgcatcc caacttgtgt tttcactatt 1320
catgggtagc agaagaccac cagggtgctc agcacactgc aaatcgtgtt ttctccttaa 1380
gtggaagtta cattcacctg gagcctgtgg ctggtaccct gccctgtggc cacacggaga 1440
ctatcacggc acactataca ctgaatagac aggccatggg agagttatcg gagctcagtt 1500
tccattacct gatcatggct aagggagtca tcgtcagatc tggaacccac actctgcctg 1560
tggagtcagg agacatgaaa ggcagttttg ccttatcctt ccctgtggag tcagacgttg 1620

cccccattgc acgaatgttc atctttgcca ttttaccaga tggagaagtt gttggagact 1680
ctgaaaaatt tgagattgaa aactgtctag ccaacaaggt ggatttgagc ttcagcccag 1740
cacaaagtcc cccagcctca catgcccacc tgcaagtagc agctgctccg cagtccctct 1800
gtgcccttcg tgctgtggac caaagtgtgc tgctcatgaa gcctgaggct gagctctctg 1860
tgtcctcagt atataatctg ctaactgtga aggatctcac caatttcct gacaatgtgg 1920
accagcagga ggaagaacaa ggacactgtc cccgtccttt cttcattcat aatggagcca 1980
tctatgttcc cttatcaagt aatgaagcag atatttatag cttcctcaag gggatgggat 2040
tgaaggtgtt cactaactca aaaatccgaa aaccaaagtc gtgttcagtc atcccttccg 2100
tgtctgcagg agcagtaggt caaggatact atggagcagg tctaggagta gtagagagac 2160
catatgttcc tcaattaggc acatataatg tgataccctt aaataatgaa caaagttcag 2220
ggccagtccc tgaaacggtg cgaagctatt ttcctgagac ttggatctgg gagttggtgg 2280
cagtgaactc atcaggtgtg gctgaggtag gagtaacagt ccctgacacc atcaccgagt 2340
ggaaggcagg ggccttctgc ctgtccgaag atgctggact tggtatctct tccactgcct 2400
ctctccgagc cttccagccc ttctttgtgg agctcacaat gccttactct gtgattcgtg 2460
gagaggtctt cacactcaag gccacggtcc taaactacct tcccaaatgc atccgggtca 2520
gtgtgcagct gaaagcctct ccagccttcc tagcttccca aaatacaaag ggagaagaat 2580
cctattgtat ctgtggaaat gagagacaaa ccttgtcttg gacagtgact cctaaaactc 2640
tggggaatgt gaacttctca gtgagtgcag aggcaatgca gtccttagaa ctctgtggaa 2700
atgaggttgt tgaggtccct gagattaaaa gaaaagacac agtcatcaaa accctgttgg 2760
tggaggctga aggtattgag caagaaaaga ctttcagttc tatgacctgt gcctcaggtg 2820
ctaatgtgtc tgagcagttg tccttgaagc tcccatcaaa tgtggtcaaa gaatctgcca 2880
gagcttcttt ctcagttctg ggtgacatat taggttctgc tatgcaaaat atacaaaatc 2940
tcctccagat gccatatggc tgtggagaac agaacatggt cctatttgct cctaacatct 3000
atgtcttgaa ctatctgaat gaaacccagc agctgacgca ggagatcaag gccaaggccg 3060
ttggctatct catcactggt taccagagac agctgaacta caaacaccaa gatggctcct 3120
acagcacctt tggggaacga tatggcagga accagggcaa cacttggctc acagcttttg 3180
tactgaagac tttcgcccag gctcgatcct acatcttcat tgatgaagca cacattaccc 3240
aatctctcac gtggctctcc cagatgcaga aggacaatgg ctgtttcagg agctctgggt 3300
cactgctcaa caatgccata aagggaggtg tagaagatga agcgaccctc tccgcctatg 3360
ttactattgc ccttctggaa attcctctcc cagtcactaa ccctattgtt cgcaatgccc 3420
tgttctgcct ggagtcagcc tggaatgtag caaaggaggg gacccatggg agccatgtct 3480
acaccaaggc attgctggcc tatgcttttt ccctactggg aaagcaaaat cagaatagag 3540
aaatactgaa ctcacttgat aaggaagctg tgaaagaaga caacctcgtc cattgggagc 3600
gccctcagag acccaaggca ccagtggggc atctttacca aacccaggct ccctctgctg 3660
aggtggagat gacatcctat gtgctcctcg cttatctcac ggcccagcca gcccccacct 3720
caggggacct gacctctgca actaacattg tgaagtggat catgaagcag cagaacgccc 3780
aaggtggttt ctcctccacc caggacacag tggtggctct ccatgccctg tccaggtatg 3840
gagcagccac tttcaccaga actgagaaaa ctgcacaggt caccgttcag gattcacaga 3900
ccttttctac aaatttccaa gtagacaaca acaacctcct attactgcag cagatctcat 3960
tgccagagct ccctggagaa tatgtcataa cagtaactgg ggaaagatgt gtgtatcttc 4020
agacatccat gaaatacaat attcttccag agaaagagga ctccccattt gctttaaaag 4080
tgcagactgt gccccaaact tgcgatggac acaaagccca caccagcttt cagatctcac 4140
tgaccatcag ttacacagga aaccgtcctg cttccaatat ggtgattgtt gatgtaaaga 4200
tggtatctgg ttttattccc ctgaaaccaa cagtaaaaat gcttgaaaga tctagctctg 4260
tgagccggac agaagtgagc aacaaccatg tcctcattta tgtggaacag gtgacaaatc 4320
agacgctaag tttttccttc atggttctgc aagacatccc agtaggagac ttgaagccag 4380
caattgttaa agtctatgat tactatgaga cagatgagtc tgtggttgct gagtatatcg 4440
ccccctgcag cacagataca gagcatggaa atgtttgagg accatacagg ctgtatattt 4500
tggtggattc tctgtcctat acatttactt agaaggaatg gagttatttg tctctataaa 4560
atagacacta aaaatatttg ctgaataaat atgtacttct ggtcaaacta aaaaa 4615
ia g[?] [?]agcurymkswbdhvnmenos[?]RNA[?] [?] [?]€[?] [?]NCBI/NM_002864
2009-09-03[?]1[?]4615 [?] €[?] <tR [?]€[?] [?]V3.3[?]CRP[?]homo
sapiensÿ8
aaggcaagag atctaggact tctagcccct gaactttcag ccgaatacat cttttccaaa 60
ggagtgaatt caggcccttg tatcactggc agcaggacgt gaccatggag aagctgttgt 120
gtttcttggt cttgaccagc ctctctcatg cttttggcca gacagacatg tcgaggaagg 180
cttttgtgtt tcccaaagag tcggatactt cctatgtatc cctcaaagca ccgttaacga 240
agcctctcaa agccttcact gtgtgcctcc acttctacac ggaactgtcc tcgacccgtg 300
ggtacagtat tttctcgtat gccaccaaga gacaagacaa tgagattctc atattttggt 360
ctaaggatat aggatacagt tttacagtgg gtgggtctga aatattattc gaggttcctg 420
aagtcacagt agctccagta cacatttgta caagctggga gtccgcctca gggatcgtgg 480
agttctgggt agatgggaag cccagggtga ggaagagtct gaagaaggga tacactgtgg 540
gggcagaagc aagcatcatc ttggggcagg agcaggattc cttcggtggg aactttgaag 600
gaagccagtc cctggtggga gacattggaa atgtgaacat gtgggacttt gtgctgtcac 660

cagatgagat taacaccatc tatcttggcg ggcccttcag tcctaatgtc ctgaactggc 720
gggcactgaa gtatgaagtg caaggcgaag tgttcaccaa accccagctg tggccctgag 780
gcccagctgt gggtcctgaa ggtacctccc ggttttttac accgcatggg ccccacgtct 840
ctgtctctgg tacctcccgc tttttacac tgcatggttc ccacgtctct gtctctgggc 900
ctttgttccc ctatatgcat tgcaggcctg ctccaccctc ctcagcgcct gagaatggag 960
gtaaagtgtc tggtctggga gctcgttaac tatgctggga aacggtccaa aagaatcaga 1020
atttgaggtg ttttgttttc attttattt caagttggac agatcttgga gataatttct 1080
tacctcacat agatgagaaa actaacaccc agaaaggaga aatgatgtta taaaaaactc 1140
ataaggcaag agctgagaag gaagcgctga tcttctattt aattccccac ccatgacccc 1200
cagaaagcag gagggcattg cccacattca cagggctctt cagtctcaga atcaggacac 1260
tggccaggtg tctggtttgg gtccagagtg ctcatcatca tgtcatagaa ctgctgggcc 1320
caggtctcct gaaatgggaa gcccagcaat accacgcagt ccctccactt tctcaaagca 1380
cactggaaag gccattagaa ttgccccagc agagcagatc tgcttttttt ccagagcaaa 1440
atgaagcact aggtataaat atgttgttac tgccaagaac ttaaatgact ggtttttgtt 1500
tgcttgcagt gctttcttaa ttttatggct cttctgggaa actcctcccc ttttccacac 1560
gaaccttgtg gggctgtgaa ttctttcttc atccccgcat tcccaatata cccaggccac 1620
aagagtggac gtgaaccaca gggtgtcctg tcagaggagc ccatctccca tctccccagc 1680
tccctatctg gaggatagtt ggatagttac gtgttcctag caggaccaac tacagtcttc 1740
ccaaggattg agttatggac tttgggagtg agacatcttc ttgctgctgg atttccaagc 1800
tgagaggacg tgaacctggg accaccagta gccatcttgt ttgccacatg gagagagact 1860
gtgaggacag aagccaaact ggaagtggag gagccaaggg attgacaaac aacagagcct 1920
tgaccacgtg gagtctctga atcagccttg tctggaacca gatctacacc tggactgccc 1980
aggtctataa gccaataaag cccctgttta cttgaaaaaa aaaa 2024homy[?]
[?]agcurymkswbdhvnm gra[?]RNA[?] [?] [?]€[?] [?]NCBI/NM_000567
2009-12-13[?]1[?]2024 [?] €[?] <tR [?]€[?] [?]V3.3[?]CFHR1[?]homo
sapiensÿž[?]cagttagtac actgaaattc aaagtcatgc tcataactgt taatgaaagc agattcaaag
60
caacaccacc accactgaag tatttttagt tatataagat tggaactacc aagcatgtgg 120
ctcctggtca gtgtaattct aatctcacgg atatcctctg ttgggggaga agcaacattt 180
tgtgattttc caaaaataaa ccatggaatt ctatatgatg aagaaaaata taagccattt 240
tcccaggttc ctacagggga agttttctat tactcctgtg aatataattt tgtgtctcct 300
tcaaaatcat tttggactcg cataacatgc acagaagaag gatggtcacc aacaccaaag 360
tgtctcagac tgtgtttctt tccttttgtg gaaaatggtc attctgaatc ttcaggacaa 420
acacatctgg aaggtgatac tgtgcaaatt atttgcaaca caggatacag acttcaaaac 480
aatgagaaca acatttcatg tgtagaacgg ggctggtcca cccctcccaa atgcaggtcc 540
actgacactt cctgtgtgaa tccgcccaca gtacaaaatg ctcatatact gtcgagacag 600
atgagtaaat atccatctgg tgagagagta cgttatgaat gtaggagccc ttatgaaatg 660
tttggggatg aagaagtgat gtgtttaaat ggaaactgga cagaaccacc tcaatgcaaa 720
gattctacgg gaaaatgtgg gcccctcca cctattgaca atggggacat tacttcattc 780
ccgttgtcag tatatgctcc agcttcatca gttgagtacc aatgccagaa cttgtatcaa 840
cttgagggta acaagcgaat aacatgtaga aatggacaat ggtcagaacc accaaaatgc 900
ttacatccgt gtgtaatatc ccgagaaatt atggaaaatt ataacatagc attaaggtgg 960
acagccaaac agaagcttta tttgagaaca ggtgaatcag ctgaatttgt gtgtaaacgg 1020
ggatatcgtc tttcatcacg ttctcacaca ttgcgaacaa catgttggga tgggaaactg 1080
gagtatccaa cttgtgcaaa aagatagaat caatcataaa atgcacacct ttattcagaa 1140
ctttagtatt aaatcagttc ttaatttcat ttttaagtat tgttttactc ctttttattc 1200
atacgtaaaa ttttggatta atttgtgaaa atgtaattat aagctgagac cggtggctct 1260
cttcttaaaa gcaccatatt aaaacttgga aaactaaaaa aaaaaaaaaa aa 1312
hata[?] [?]agcurymkswbdhvnminus[?]RNA[?] [?] [?]€[?] [?]NCBI/NM_002113
2009-12-13[?]1[?]1312 [?] €[?] <tR [?]€[?]
[?]V3.3[?]SERPINA3[?]homo sapiensÿj[?]attcatgaaa atccactact ccagacagac ggctttggaa tccaccagct
acatccagct 60
ccctgaggca gagttgagaa tggagagaat gttacctctc ctggctctgg ggctcttggc 120
ggctgggttc tgccctgctg tcctctgcca ccctaacagc ccacttgacg aggagaatct 180
gacccaggag aaccaagacc gagggacaca cgtggacctc ggattagcct ccgccaacgt 240
ggacttcgct ttcagcctgt acaagcagtt agtcctgaag gcccctgata agaatgtcat 300
cttctcccca ctgagcatct ccaccgcctt ggccttcctg tctctggggg cccataatac 360
caccctgaca gagattctca aaggcctcaa gttcaacctc acggagactt ctgaggcaga 420
aattcaccag agcttccagc acctcctgcg caccctcaat cagtccagcg atgagctgca 480
gctgagtatg ggaaatgcca tgtttgtcaa agagcaactc agtctgctgg acaggttcac 540
ggaggatgcc aagaggctgt atggctccga ggcctttgcc actgactttc aggactcagc 600
tgcagctaag aagctcatca acgactacgt gaagaatgga actaggggga aaatcacaga 660
tctgatcaag gaccttgact cgcagacaat gatggtcctg gtgaattaca tcttctttaa 720
agccaaatgg gagatgccct ttgaccccca agatactcat cagtcaaggt tctacttgag 780

caagaaaaag tgggtaatgg tgcccatgat gagtttgcat cacctgacta taccttactt 840
ccgggacgag gagctgtcct gcaccgtggt ggagctgaag tacacaggca atgccagcgc 900
actcttcatc ctccctgatc aagacaagat ggaggaagtg gaagccatgc tgctcccaga 960
gaccctgaag cggtggagag actctctgga gttcagagag ataggtgagc tctacctgcc 1020
aaagttttcc atctcgaggg actataacct gaacgacata cttctccagc tgggcattga 1080
ggaagccttc accagcaagg ctgacctgtc agggatcaca ggggccagga acctagcagt 1140
ctcccaggtg gtccataagg ctgtgcttga tgtatttgag gagggcacag aagcatctgc 1200
tgccacagca gtcaaaatca ccctcctttc tgcattagtg gagacaagga ccattgtgcg 1260
tttcaacagg cccttcctga tgatcattgt ccctacagac acccagaaca tcttcttcat 1320
gagcaaagtc accaatccca agcaagccta gagcttgcca tcaagcagtg gggctctcag 1380
taaggaactt ggaatgcaag ctggatgcct gggtctctgg gcacagcctg gcccctgtgc 1440
accgagtggc catggcatgt gtggccctgt ctgcttatcc ttggaaggtg acagcgattc 1500
cctgtgtagc tctcacatgc acaggggccc atggactctt cagtctggag ggtcctgggc 1560
ctcctgacag caataaataa tttcgttgga aaaaaaaaaa aaaaaaaaaa aaaaaaaaaa 1620
aaaaaaaaa 1629trid[?]
[?]agcurymkswbdhvnmClos[?]RNA[?] [?] [?]€[?] [?]NCBI/NM_001085
2009-12-06[?]1[?]1629 [?] €[?] <tR [?]€[?] [?]V3.3[?]HABP2[?]homo
sapiensÿU[?]atttgttcct gaatccttgg agactgacat tttccccccc taaaggcata gacaacaaaa
60
gaaattttat tgagaggaaa acacaagtcc ttaaactgca aagatgtttg ccaggatgtc 120
tgatctccat gttctgctgt taatggctct ggtgggaaag acagcctgtg ggttctccct 180
gatgtcttta ttggaaagcc tggacccaga ctggacccct gaccagtatg attacagcta 240
cgaggattat aatcaggaag agaacaccag tagcacactt acccacgctg agaatcctga 300
ctggtactac actgaggacc aagctgatcc atgccagccc aacccctgtg aacacggtgg 360
ggactgcctc gtccatggga gcaccttcac atgcagctgc ctggctcctt tctctgggaa 420
taagtgtcag aaagtgcaaa atacgtgcaa ggacaaccca tgtggccggg gccaatgtct 480
cattacccag agtcctccct actaccgctg tgtctgtaaa caccccttaca caggtcccag 540
ctgctcccaa gtggttcctg tatgcaggcc aaacccctgc cagaatgggg ctacctgctc 600
ccggcataag cggagatcca agttcacctg tgcctgtccc gaccagttca aggggaaatt 660
ctgtgaaata ggttctgatg actgctatgt tggcgatggc tactcttacc gagggaaaat 720
gaataggaca gtcaaccagc atgcgtgcct ttactggaac tcccacctcc tcttgcagga 780
gaattacaac atgtttatgg aggatgctga aacccatggg attggggaac acaatttctg 840
cagaaaccca gatgcggacg aaaagccctg gtgctttatt aaagttacca atgacaaggt 900
gaaatgggaa tactgtgatg tctcagcctg ctcagcccag gacgttgcct acccagagga 960
aagccccact gagccatcaa ccaagcttcc ggggtttgac tcctgtggaa agactgagat 1020
agcagagagg aagatcaaga gaatctatgg aggctttaag agcacggcgg gcaagcaccc 1080
atggcaggcg tccctccagt cctcgctgcc tctgaccatc tccatgcccc agggccactt 1140
ctgtggtggg gcgctgatcc accccctgctg ggtgctcact gctgcccact gcaccgacat 1200
aaaaaccaga catctaaagg tggtgctagg ggaccaggac ctgaagaaag aagaatttca 1260
tgagcagagc tttagggtgg agaagatatt caagtacagc cactacaatg aaagagatga 1320
gattccccac aatgatattg cattgctcaa gttaaagcca gtggatggtc actgtgctct 1380
agaatccaaa tacgtgaaga ctgtgtgctt gcctgatggg tcctttccct ctgggagtga 1440
gtgccacatc tctggctggg gtgttacaga aacaggaaaa gggtcccgcc agctcctgga 1500
tgccaaagtc aagctgattg ccaacacttt gtgcaactcc cgccaactct atgaccacat 1560
gattgatgac agtatgatct gtgcaggaaa tcttcagaaa cctgggcaag acacctgcca 1620
gggtgactct ggaggccccc tgacctgtga gaaggacggc acctactacg tctatgggat 1680
agtgagctgg ggcctggagt gtgggaagag gccaggggtc tacacccaag ttaccaaatt 1740
cctgaattgg atcaaagcca ccatcaaaag tgaaagtggc ttctaaggta ctgtcttctg 1800
gacctcagag cccactctcc ttggcaccct gacaccggga ggcctcatgg ccaacaatgg 1860
acacctccag agcctccagg ggaccacaca gtagactatc cctactctaa gcagagacaa 1920
ctgccaccca gcctgggcct tcccagacca gcatttgcac aatatcacca ggcttcttct 1980
gcctcccttg gtaacccaag gaatgatgga atcaacacaa catagtatgt ttgctttcct 2040
tacccaattg taccttctag aaaatcagtg ttcacagaga ctgcctccac cacaggcatc 2100
ctgcaaatgc agactccaga atccccagca tcagcgggaa ccaccatcac atctttattc 2160
ctcagcccag acactcgagg cactcaacag aatcagccat ccacgtctag gtatcagaga 2220
ggaccacaaa tacaacattc tccatctgct ttcagagtta ttattttaat aaaggaagat 2280
ctgggatggg ctggtgggcc attccagctt gccgaaatca aagccatctg aagcctgtct 2340
ctggtgaaca aacttcctct ctggcctctc aggaatcagg gtggacatgg ctcacaacag 2400
cagggccttc ttctttttga cgtgcagaat ctcagtggca tctgggttca cctccccact 2460
ctgatgatct ccagcctcca ctgcttctgc cccccgctgc tgaaatcaaa cataccccaa 2520
gttaaaatga agctccccca cccccactcc cggccccggt tcccacagga cacgctaaga 2580
agcacaggga gcatttaaca ggctcaccct ccctttcctt ttcccctctt ctaccctccc 2640
caagaaaaag ggccttcaag gcaggaatga gaaagcaaag ccaatctctc atttagacct 2700
ggcttctttc ttctgaacaa agtagggttc aaaatgcaga ctgtcatatc cagcgagtcc 2760

ctgacccttt ctgcgaatgt aacgagcaag cagtcagcac agcctgggct gccctggccc 2820
gggattgatg tagccccggt aggtttgcct ctgcagaact aatggctgtg acttcagaga 2880
aagccctgca ggaagtttaa cctgcgtgtc atctgcctgg tcatctcaga cccatgaaat 2940
taggcgcctt gtttgagctg cgtttcacac ttctttagag ctagctgacc tttggccaaa 3000
aataaacttt gaaaagaaa 3019x[?] [?][?]
[?]agcurymkswbdhvnm<tR [?]RNA[?] [?] [?]€ [?]NCBI/NM_004132
2009-12-07[?]1[?]3019 [?] € <tR [?]€[?] [?]V3.3[?]CNDP1[?]homo
sapiensÿ![?]gcagttgagc tgaatgaata cctccgaagc cgctttgttc tccagatgtg aatagctcca
60
ctataccagc ctcgtcttcc ttccggggga caacgtgggt cagggcacag agagatattt 120
aatgtcaccc tcttggggct ttcatgggac tccctctgcc acatttttg gaggttggga 180
aagttgctag aggcttcaga actccagcct aatggatccc aaactcggga gaatggctgc 240
gtccctgctg gctgtgctgc tgctgctgct ggagcgcggc atgttctcct caccctcccc 300
gcccccggcg ctgttagaga aagtcttcca gtacattgac ctccatcagg atgaatttgt 360
gcagacgctg aaggagtggg tggccatcga gagcgactct gtccagcctg tgcctcgctt 420
cagacaagag ctcttcagaa tgatggccgt ggctgcggac acgctgcagc gcctgggggc 480
ccgtgtggcc tcggtggaca tgggtcctca gcagctgccc gatggtcaga gtcttccaat 540
acctcccgtc atcctggccg aactggggag cgatcccacg aaaggcaccg tgtgcttcta 600
cggccacttg gacgtgcagc ctgctgaccg gggcgatggg tggctcacgg acccctatgt 660
gctgacggag gtagacggga aactttatgg acgaggagcg accgacaaca aaggccctgt 720
cttggcttgg atcaatgctg tgagcgcctt cagagccctg gagcaagatc ttcctgtgaa 780
tatcaaattc atcattgagg ggatggaaga ggctggctct gttgccctgg aggaacttgt 840
ggaaaaagaa aaggaccgat tcttctctgg tgtggactac attgtaattt cagataacct 900
gtggatcagc caaaggaagc cagcaatcac ttacggaacc cgggggaaca gctacttcat 960
ggtggaggtg aaatgcagag accaggattt tcactcagga acctttggtg gcatccttca 1020
tgaaccaatg gctgatctgg ttgctcttct cggtagcctg gtagactcgt ctggtcatat 1080
cctggtccct ggaatctatg atgaagtggt tcctcttaca gaagaggaaa taaatacata 1140
caaagccatc catctagacc tagaagaata ccggaatagc agccgggttg agaaatttct 1200
gttcgatact aaggaggaga ttctaatgca cctctggagg tacccatctc tttctattca 1260
tgggatcgag ggcgcgtttg atgagcctgg aactaaaaca gtcatacctg gccgagttat 1320
aggaaaattt tcaatccgtc tagtccctca catgaatgtg tctgcggtgg aaaaacaggt 1380
gacacgacat cttgaagatg tgttctccaa aagaaatagt tccaacaaga tggttgtttc 1440
catgactcta ggactacacc cgtggattgc aaatattgat gacacccagt atctcgcagc 1500
aaaaagagcg atcagaacag tgtttggaac agaaccagat atgatccggg atggatccac 1560
cattccaatt gccaaaatgt tccaggagat cgtccacaag agcgtggtgc taattccgct 1620
gggagctgtt gatgatggag aacattcgca gaatgagaaa atcaacaggt ggaactacat 1680
agagggaacc aaattatttg ctgccttttt cttagagatg gcccagctcc attaatcaca 1740
agaaccttct agtctgatct gatccactga cagattcacc tcccccacat ccctagacag 1800
ggatggaatg taaatatcca gagaatttgg gtctagtata gtacattttc ccttccattt 1860
aaaatgtctt gggatatctg gatcagtaat aaaatatttc aaaggcacag atgttggaaa 1920
tggtttaagg tcccccactg cacaccttcc tcaagtcata gctgcttgca gcaacttgat 1980
ttccccaagt cctgtgcaat agccccagga ttggattcct tcaaaccttt tagcatatct 2040
ccaaccttgc aatttgattg gcataatcac tccagtttgc tttctaggtc ctcaagtgct 2100
cgtgacacat aatcattcca tccaatgatc gcctttgctt taccactctt tccttttatc 2160
ttattaataa aaatgttggt ctccaccact gactacaaaa aaaaaaaaaa a 2211
[?]kÉ [?] [?]agcurymkswbdhvnm ctg[?]RNA[?] [?] [?]€# [?]NCBI/NM_032649
2009-09-03[?]1[?]2211 [?] €# sum [?]€[?] [?]V3.3[?]CFHR2[?]homo
sapiensÿh[?]tgttaatgaa agcagattca aagcaacacc accaccactg aagtattttt agttatataa
60
gattggaact accaagcatg tggctcctgg tcagtgtaat tctaatctca cggatatcct 120
ctgttggggg agaagcaatg ttctgtgatt ttccaaaaat aaaccatgga attctatatg 180
atgaagaaaa atataagcca ttttcccaag ttcctacagg ggaagttttc tattactcct 240
gtgaatataa ttttgtgtct ccttcaaaat ccttttggac tcgcataacg tgcgcagaag 300
aaggatggtc accaacacca aagtgtctca gactgtgttt ctttcctttt gtggaaaatg 360
gtcattctga atcttcagga caaacacatc tggaaggtga tactgtacaa attatttgca 420
acacaggata cagacttcaa aacaatgaga acaacatttc atgtgtagaa cggggctggt 480
ccactcctcc caaatgcagg tccactattt ctgcagaaaa atgtgggccc cctccaccta 540
ttgacaatgg agacattact tcattcctgt tgtcagtata tgctccaggt tcatcagttg 600
agtaccagtg ccagaacttg tatcaacttg agggtaacaa tcaaataaca tgtagaaacg 660
gacaatggtc agaaccacca aaatgcttag atccatgtgt aatatcacaa gaaattatgg 720
aaaaatataa cataaaatta aagtggacaa accaacaaaa gctttattca agaacaggtg 780
acatagttga atttgtttgt aaatctggat atcatccaac aaaatctcat tcatttcgag 840
caatgtgtca gaatggaaaa ctggtatatc ccagttgtga agaaaaatag aatcaatggc 900
attactatta gtaaaatgca cacctttttc tgaatttact attatatttg ttttcaattt 960

cattttcaa gtactgtttt actcattttt attcataaat aaagttttgt gttgatttgt 1020
gaaaatgcaa ttacaagagc caa 1043aatg[?]
[?]agcurymkswbdhvnm ggg[?]RNA[?] [?] [?]€& [?]NCBI/NM_005666
2009-09-03[?]1[?]1043 [?] €& <tR [?]€[?] [?]V3.3[?]SAA[?]homo
sapiensÿ [?]agggacccgc agctcagcta cagcacagat cagcaccatg aagcttctca cgggcctggt
60
tttctgctcc ttggtcctga gtgtcagcag ccgaagcttc ttttcgttcc ttggcgaggc 120
ttttgatggg gctcgggaca tgtggagagc ctactctgac atgagagaag ccaattacat 180
cggctcagac aaatacttcc atgctcgggg gaactatgat gctgccaaaa ggggacctgg 240
gggtgcctgg gccgcagaag tgatcagcaa tgccagagag aatatccaga gactcacagg 300
ccatggtgcg gaggactcgc tggccgatca ggctgccaat aaatggggca ggagtggcag 360
agaccccaat cacttccgac ctgctggcct gcctgagaaa tactgagctt cctcttcact 420
ctgctctcag gagacctggc tatgaggccc tcggggcagg gatacaaagt tagtgaggtc 480
tatgtccaga gaagctgaga tatggcatat aataggcatc taataaatgc ttaagaggtc 540
aaaaaaaaaa aaaaaaaaaa aaaaaaaaaa 570\[?]É [?]
[?]agcurymkswbdhvnm ctg[?]RNA[?] [?] [?]€)
NCBI/BC020795
2009-07-15[?]1[?]570 [?] €) <tR [?]€[?] [?]V3.3[?]PROZ[?]homo
sapiensÿƒ[?]ggtgggaatg gcaggctgcg tcccactgct ccagggcctg gtcctggtcc tcgccctcca
60
tcgtgtggag ccctcagtat ttctcccggc ctccaaagca aacgacgttc tggtgaggtg 120
gaagcgtgcg ggctcctatc ttctggaaga actcttcgag ggaaacttgg aaaaagaatg 180
ttatgaagaa atctgtgtct atgaagaagc aagagaagtg tttgaaaatg aagtagtcac 240
tgatgaattc tggagacgat ataagggcgg ctccccgtgc atctcccagc cctgcctcca 300
caacggctct tgccaggaca gcatctgggg ctacacctgc acctgctccc ccggctatga 360
gggcagcaac tgcgagctgg ctaaaaatga atgtcaccca gagcggactg atgggtgtca 420
acacttctgc ctcccaggac aggaatccta cacgtgcagc tgtgctcagg gctacaggct 480
tggtgaggac cacaaacagt gtgtgcccca cgaccagtgt gcctgcgggg tgctgacctc 540
tgagaagcgt gcaccggatc tacaggacct cccgtggcag gtaaagttaa caaattccga 600
aggaaaagac ttctgtggtg gtgttataat acgggaaaat tttgtactga caacagcaaa 660
atgttcactg ttacacagga atattactgt aaaaacatat tttaacagaa cgagccaaga 720
cccgctgatg atcaagataa cgcacgtcca tgtgcacatg cggtatgacg cggacgcggg 780
ggagaatgac ctgtcactgc tggagctgga gtggcccatc cagtgcccag gtgcggggct 840
ccccgtgtgc accccctgaga aagacttcgc tgagcacctc ctcatcccac gcaccagggg 900
cctcctcagc ggctgggcac gcaatggcac tgacctgggc aactcgctga ccacgcggcc 960
tgtcacactt gtggaggggg aggagtgcgg gcaggtcctg aatgtgactg tcaccaccag 1020
gacctactgt gagagaagca gcgtggcggc catgcactgg atggatggaa gtgtggtcac 1080
cagagaacac agaggctcct ggtttctcac gggggtcctg ggctcgcagc cagtaggagg 1140
gcaggctcac atggtccttg tcaccaaggt ctccaggtac tcactctggt ttaaacagat 1200
catgaactaa ctgaaactca gctagccaga atgaacaaca caaccggaag cgggattcca 1260
agctggcact gccactgtgg agggcgctga aacttcatca cacactgaga ggccgtcaca 1320
gccccagacc acccgcttgg cccacgcagc agcagagccg ccgtttgctg ggttgtttac 1380
cgagcactgt gacctttctt tccctggaac tctttatctc aatagagacc ttaaaagaaa 1440
acatgagata cgttaaataa taaaataaga taatctgtca gtcat 1485gt g[?]
[?]agcurymkswbdhvnm [?]RNA[?] [?] [?]€, [?]NCBI/NM_003891
2009-12-06[?]1[?]1485 [?] €, <tR [?]€[?] [?]V3.3[?]PON3[?]homo
sapiensÿO[?]agtcgccgct gggcaccccg agaccatggg gaagctcgtg gcgctggtcc tgctgggggt
60
cggcctgtcc ttagtcgggg agatgttcct ggcgtttaga gaaagggtga atgcctctcg 120
agaagtggag ccagtagaac ctgaaaactg ccaccttatt gaggaacttg aaagtggctc 180
tgaagatatt gatatacttc ctagtgggct ggcttttatc tccagtggat taaaatatcc 240
aggcatgcca aactttgcgc cagatgaacc aggaaaaatc ttcttgatgg atctgaatga 300
acaaaaccca agggcacaag cgctagaaat cagtggtgga tttgacaaag aattatttaa 360
tccacatggg atcagtattt tcatcgacaa agacaatact gtgtatcttt atgttgtgaa 420
tcatccccac atgaagtcca ctgtggagat atttaaattt gaggaacaac aacgttctct 480
ggtatacctg aaaactataa aacatgaact tctcaaaagt gtgaatgaca ttgtggttct 540
tggaccagaa cagttctatg ccaccagaga ccactatttt accaactccc tcctgtcatt 600
ttttgagatg atcttggatc ttcgctggac ttatgttctt ttctacagcc caagggaggt 660
taaagtggtg gccaaaggat tttgtagtgc caatgggatc acagtctcag cagaccagaa 720
gtatgtctat gtagctgatg tagcagctaa gaacattcac ataatggaaa aacatgataa 780
ctgggattta actcaactga aggtgataca gttgggcacc ttagtggata acctgactgt 840
cgatcctgcc acaggagaca ttttggcagg atgccatcct aatcctatga agctactgaa 900
ctataaccct gaggaccctc caggatcaga agtacttcgc atccagaatg ttttgtctga 960
gaagcccagg gtgagcaccg tgtatgccaa caatggctct gtgcttcagg gcacctctgt 1020

fc712142009.txt
ggcttctgtg taccatggga aaattctcat aggcaccgta tttcacaaaa ctctgtactg 1080
tgagctctag actctagata gtaaaaaaaa aaaaaaaaag tctacatatt ttgtaaaagt 1140
aaactgataa ttgtatgata agtggcactg taagtaaata gcaaacacca aaaaaaaaaa 1200
a 12010
g[?] [?]agcurymkswbdhvnmcaca[?]RNA[?] [?] [?]€/ [?]NCBI/NM_000940
2009-12-13[?]1[?]1201 [?] €/ <tR [?]€[?]
[?]V3.3[?]RARRES2[?]homo sapiensÿç[?]gccgccccgc gagaagaaga gcgggaagag gcggacagcg aggccaagat
ttcagctgcg 60
ggacggtcag gggagacctc caggcgcagg gaaggacggc cagggtgaca cggaagcatg 120
cgacggctgc tgatccctct ggccctgtgg ctgggtgcgg tgggcgtggg cgtcgccgag 180
ctcacggaag cccagcgccg gggcctgcag gtggccctgg aggaatttca caagcacccg 240
cccgtgcagt gggccttcca ggagaccagt gtggagagcg ccgtggacac gcccttccca 300
gctggaatat ttgtgaggct ggaatttaag ctgcagcaga caagctgccg gaagagggac 360
tggaagaaac ccgagtgcaa agtcaggccc aatgggagga aacggaaatg cctggcctgc 420
atcaaactgg gctctgagga caaagttctg ggccggttgg tccactgccc catagagacc 480
caagttctgc gggaggctga ggagcaccag gagacccagt gcctcagggt gcagcgggct 540
ggtgaggacc cccacagctt ctacttccct ggacagttcg ccttctccaa ggccctgccc 600
cgcagctaag ccagcactga gatgcgtggt gcctccagga ccgctgcggg tggtaaccag 660
tggaagaccc cagcccccag ggagaggaac ccgttctatc cccagccatg ataataaagc 720
tgctctccca gctgcctctc aaaaaaaaaa aaaaaaaaaa aaaaaaa 767cc t[?]
[?]agcurymkswbdhvnmttcc[?]RNA[?] [?] [?]€2 [?]NCBI/NM_002889
2009-12-07[?]1[?]767 [?] €2 [?]
[?]€[?] [?]V3.3[?]GGH[?]homo sapiensÿÐ[?]cagagtctcc gcttaggcgg cccgtggggg tcggggtcgc
ggtcgcggtc gcggaccggg 60
gacagagcaa tggggcgggc gcgcagcgcc ggctgcactc ccagagcctc ccaccgccgc 120
cccacggccg cctagcgcgc gccgggactg catttcccat aggcccacgc tgcggagcac 180
cgcccacccg agtctcacgc gagtgcgctg ccgcgaggtg tccctgccgc agcccccgcc 240
cgcccgcaga gcttttgaaa ggcggcggga ggcggcgagc gccatggcca gtccgggctg 300
cctgctgtgc gtgctgggcc tgctactctg cggggcggcg agcctcgagc tgtctagacc 360
ccacggcgac accgccaaga agcccatcat cggaatatta atgcaaaaat gccgtaataa 420
agtcatgaaa aactatggaa gatactatat tgctgcgtcc tatgtaaagt acttggagtc 480
tgcaggtgcg agagttgtac cagtaaggct ggatcttaca gagaaagact atgaaatact 540
tttcaaatct attaatggaa tccttttccc tggaggaagt gttgacctca gacgctcaga 600
ttatgctaaa gtggccaaaa tattttataa cttgtccata cagagttttg atgatggaga 660
ctattttcct gtgtggggca catgccttgg atttgaagag ctttcactgc tgattagtgg 720
agagtgctta ttaactgcca cagatactgt tgacgtggca atgccgctga acttcactgg 780
aggtcaattg cacagcagaa tgttccagaa ttttcctact gagttgttgc tgtcattagc 840
agtagaacct ctgactgcca atttccataa gtggagcctc tccgtgaaga attttacaat 900
gaatgaaaag ttaaagaagt ttttcaatgt cttaactaca aatacagatg gcaagattga 960
gtttatttca acaatggaag gatataagta tccagtatat ggtgtccagt ggcatccaga 1020
gaaagcacct tatgagtgga agaatttgga tggcatttcc catgcaccta atgctgtgaa 1080
aaccgcattt tatttagcag agtttttgt taatgaagct cggaaaaaca accatcattt 1140
taaatctgaa tctgaagagg agaaagcatt gatttatcag ttcagtccaa tttatactgg 1200
aaatatttct tcatttcagc aatgttacat atttgattga aagtcttcaa tttgttaaca 1260
gagcaaattt gaataattcc atgattaaac tgttagaata acttgctact catggcaaga 1320
ttaggaagtc acagattctt ttctataatg tgcctggctc tgattcttca ttctgtatgt 1380
gactatttat ataacattag ataattaaat agtgagacat aaatagagtg tttttcatgg 1440
aaaagccttc ttatatctga agattgaaaa aaataaattt actgaaatac aaaaaaaaaa 1500
aaaaa 1505DOÈ [?]
[?]agcurymkswbdhvnmaaat[?]RNA[?] [?] [?]€5 [?]NCBI/NM_003878
2009-12-07[?]1[?]1505 [?] €5 <tR [?]€[?] [?]V3.3[?]PRG4[?]homo
sapeinsÿ[?][?]aaactcatct atcctttacg gcaagggtac ctacggtacc tgaaaacaac gatggcatgg
60
aaaacacttc ccatttacct gttgttgctg ctgtctgttt tcgtgattca gcaagtttca 120
tctcaagatt tatcaagctg tgcagggaga tgtggggaag ggtattctag agatgccacc 180
tgcaactgtg attataactg tcaacactac atggagtgct gccctgattt caagagagtc 240
tgcactgcgg agctttcctg taaaggccgc tgctttgagt ccttcgagag agggagggag 300
tgtgactgcg acgcccaatg taagaagtat gacaagtgct gtcccgatta tgagagtttc 360
tgtgcagaag tgcataatcc cacatcacca ccatcttcaa agaaagcacc tccaccttca 420
ggagcatctc aaaccatcaa atcaacaacc aaacgttcac ccaaaccacc aaacaagaag 480
aagactaaga aagttataga atcagaggaa ataacagaag aacattctgt ttctgaaaat 540
caagagtcct cctcctcctc ctcctcttcc tcttcttctt caacaattcg gaaaatcaag 600
tcttccaaaa attcagctgc taatagagaa ttacagaaga aactcaaagt aaaagataac 660
aagaagaaca gaactaaaaa gaaacctacc cccaaaccac cagttgtaga tgaagctgga 720

agtggattgg acaatggtga cttcaaggtc acaactcctg acacgtctac cacccaacac 780
aataaagtca gcacatctcc caagatcaca acagcaaaac caataaatcc cagacccagt 840
cttccaccta attctgatac atctaaagag acgtctttga cagtgaataa agagacaaca 900
gttgaaacta aagaaactac tacaacaaat aaacagactt caactgatgg aaaagagaag 960
actacttccg ctaaagagac acaaagtata gagaaaacat ctgctaaaga tttagcaccc 1020
acatctaaag tgctggctaa acctacaccc aaagctgaaa ctacaaccaa aggccctgct 1080
ctcaccactc ccaaggagcc cacgcccacc actcccaagg agcctgcatc taccacaccc 1140
aaagagccca cacctaccac catcaagtct gcacccacca cccccaagga gcctgcaccc 1200
accaccacca agtctgcacc caccactccc aaggagcctg cacccaccac caccaaggag 1260
cctgcaccca ccactcccaa ggagcctgca cccaccacca ccaaggagcc tgcacccacc 1320
accaccaagt ctgcacccac cactcccaag gagcctgcac ccaccacccc caagaagcct 1380
gccccaacta cccccaagga gcctgcaccc accactccca aggagcctac acccaccact 1440
cccaaggagc ctgcacccac caccaaggag cctgcaccca ccactcccaa agagcctgca 1500
cccactgccc ccaagaagcc tgccccaact acccccaagg agcctgcacc caccactccc 1560
aaggagcctg cacccaccac caccaaggag ccttcaccca ccactcccaa ggagcctgca 1620
cccaccacca ccaagtctgc acccaccact accaaggagc ctgcacccac cactaccaag 1680
tctgcaccca ccactcccaa ggagccttca cccaccacca ccaaggagcc tgcacccacc 1740
actcccaagg agcctgcacc caccaccccc aagaagcctg ccccaactac ccccaaggag 1800
cctgcaccca ccactcccaa ggaacctgca cccaccacca ccaagaagcc tgcacccacc 1860
actcccaaag agcctgcccc aactaccccc aaggagactg cacccaccac ccccaagaag 1920
ctcacgccca ccacccccga gaagctcgca cccaccaccc ctgagaagcc cgcacccacc 1980
acccctgagg agctcgcacc caccacccct gaggagccca cacccaccac ccctgaggag 2040
cctgctccca ccactcccaa ggcagcggct cccaacaccc ctaaggagcc tgctccaact 2100
acccctaagg agcctgctcc aactacccct aaggagcctg ctccaactac ccctaaggag 2160
actgctccaa ctacccctaa agggactgct ccaactaccc tcaaggaacc tgcacccact 2220
actcccaaga agcctgcccc caaggagctt gcacccacca ccaccaagga gcccacatcc 2280
accacctgtg acaagcccgc tccaactacc cctaagggga ctgctccaac taccccctaag 2340
gagcctgctc caactacccc taaggagcct gctccaacta cccctaaggg gactgctcca 2400
actaccctca aggaacctgc acccactact cccaagaagc ctgcccccaa ggagcttgca 2460
cccaccacca ccaaggggcc cacatccacc acctctgaca agcctgctcc aactacacct 2520
aaggagactg ctccaactac ccccaaggag cctgcaccca ctacccccaa gaagcctgct 2580
ccaactactc ctgagacacc tcctccaacc acttcagagg tctctactcc aactaccacc 2640
aaggagccta ccactatcca caaaagccct gatgaatcaa ctcctgagct ttctgcagaa 2700
cccacaccaa aagctcttga aaacagtccc aaggaacctg gtgtacctac aactaagact 2760
cctgcagcga ctaaacctga aatgactaca acagctaaag acaagacaac agaaagagac 2820
ttacgtacta cacctgaaac tacaactgct gcacctaaga tgacaaaaga gacagcaact 2880
acaacagaaa aaactaccga atccaaaata acagctacaa ccacacaagt aacatctacc 2940
acaactcaag ataccacacc attcaaaatt actactctta aaacaactac tcttgcaccc 3000
aaagtaacta caacaaaaaa gacaattact accactgaga ttatgaacaa acctgaagaa 3060
acagctaaac caaaagacag agctactaat tctaaagcga caactcctaa acctcaaaag 3120
ccaaccaaag cacccaaaaa acccacttct accaaaaagc caaaaacaat gcctagagtg 3180
agaaaaccaa agacgacacc aactccccgc aagatgacat caacaatgcc agaattgaac 3240
cctacctcaa gaatagcaga agccatgctc caaaccacca ccagacctaa ccaaactcca 3300
aactccaaac tagttgaagt aaatccaaag agtgaagatg caggtggtgc tgaaggagaa 3360
acacctcata tgcttctcag gccccatgtg ttcatgcctg aagttactcc cgacatggat 3420
tacttaccga gagtacccaa tcaaggcatt atcatcaatc ccatgctttc cgatgagacc 3480
aatatatgca atggtaagcc agtagatgga ctgactactt tgcgcaatgg gacattagtt 3540
gcattccgag gtcattattt ctggatgcta agtccattca gtccaccatc tccagctcgc 3600
agaattactg aagtttgggg tattccttcc cccattgata ctgtttttac taggtgcaac 3660
tgtgaaggaa aaactttctt ctttaaggat tctcagtact ggcgttttac caatgatata 3720
aaagatgcag ggtaccccaa accaattttc aaaggatttg gaggactaac tggacaaata 3780
gtggcagcgc tttcaacagc taaatataag aactggcctg aatctgtgta tttttcaag 3840
agaggtggca gcattcagca gtatatttat aaacaggaac ctgtacagaa gtgccctgga 3900
agaaggcctg ctctaaatta tccagtgtat ggagaaacga cacaggttag gagacgtcgc 3960
tttgaacgtg ctataggacc ttctcaaaca cacaccatca gaattcaata ttcacctgcc 4020
agactggctt atcaagacaa aggtgtcctt cataatgaag ttaaagtgag tatactgtgg 4080
agaggacttc caaatgtggt tacctcagct atatcactgc ccaacatcag aaaacctgac 4140
ggctatgatt actatgcctt ttctaaagat caatactata acattgatgt gcctagtaga 4200
acagcaagag caattactac tcgttctggg cagaccttat ccaaagtctg gtacaactgt 4260
ccttagactg atgagcaaag gaggagtcaa ctaatgaaga aatgaataat aaattttgac 4320
actgaaaaac attttattaa taaagaatat tgacatgagt ataccagttt atatataaaa 4380
atgtttttaa acttgacaat cattacacta aaacagattt gataatctta ttcacagttg 4440
ttattgttta cagaccattt aattaatatt tcctctgttt attcctcctc tccctcccat 4500
tgcatggctc acacctgtaa aagaaaaaag aatcaaattg aatatatctt ttaagaattc 4560

aaaactagtg tattcactta ccctagttca ttataaaaaa tatctaggca ttgtggatat 4620
aaaactgttg ggtattctac aacttcaatg gaaattatta caagcagatt aatccctctt 4680
tttgtgacac aagtacaatc taaaagttat attggaaaac atggaaatat taaaatttta 4740
cacttttact agctaaaaca taatcacaaa gctttatcgt gttgtataaa aaaattaaca 4800
atataatggc aataggtaga gatacaacaa atgaatataa cactataaca cttcatattt 4860
tccaaatctt aatttggatt taaggaagaa atcaataaat ataaaatata agcacatatt 4920
tattatatat ctaaggtata caaatctgtc tacatgaagt ttacagattg gtaaatatca 4980
cctgctcaac atgtaattat ttaataaaac tttggaacat taaaaaaata aattggaggc 5040
ttaaaaaaaa aaaaaaaaa 5059ta a[?]
[?]agcurymkswbdhvnmggct[?]RNA[?] [?] [?]€8 [?]NCBI/NM_005807
2009-06-21[?]1[?]5059 [?] €8 [?]€[?] [?]V3.3[?]MCAM[?]homo
sapiensÿÖ[?]acttggctct cgccctccgg ccgggaagca tggggcttcc caggctggtc tgcgccttct
60
tgctcgccgc ctgctgctgc tgtcctcgcg tcgcgggtgt gcccggagag gctgagcagc 120
ctgcgcctga gctggtggag gtggaagtgg gcagcacagc ccttctgaag tgcggcctct 180
cccagtccca aggcaacctc agccatgtcg actggttttc tgtccacaag gagaagcgga 240
cgctcatctt ccgtgtgcgc cagggccagg gccagagcga acctggggag tacgagcagc 300
ggctcagcct ccaggacaga ggggctactc tggccctgac tcaagtcacc ccccaagacg 360
agcgcatctt cttgtgccag ggcaagcgcc ctcggtccca ggagtaccgc atccagctcc 420
gcgtctacaa agctccggag gagccaaaca tccaggtcaa cccccctgggc atccctgtga 480
acagtaagga gcctgaggag gtcgctacct gtgtagggag gaacgggtac cccattcctc 540
aagtcatctg gtacaagaat ggccggcctc tgaaggagga gaagaaccgg gtccacattc 600
agtcgtccca gactgtggag tcgagtggtt tgtacacctt gcagagtatt ctgaaggcac 660
agctggttaa agaagacaaa gatgcccagt tttactgtga gctcaactac cggctgccca 720
gtgggaacca catgaaggag tccagggaag tcaccgtccc tgttttctac ccgacagaaa 780
aagtgtggct ggaagtggag cccgtgggaa tgctgaagga aggggaccgc gtggaaatca 840
ggtgtttggc tgatggcaac cctccaccac acttcagcat cagcaagcag aaccccagca 900
ccagggaggc agaggaagag acaaccaacg acaacggggt cctggtgctg gagcctgccc 960
ggaaggaaca cagtgggcgc tatgaatgtc agggcctgga cttggacacc atgatatcgc 1020
tgctgagtga accacaggaa ctactggtga actatgtgtc tgacgtccga gtgagtcccg 1080
cagcccctga gagacaggaa ggcagcagcc tcaccctgac ctgtgaggca gagagtagcc 1140
aggacctcga gttccagtgg ctgagagaag agacaggcca ggtgctggaa agggggcctg 1200
tgcttcagtt gcatgacctg aaacgggagg caggaggcgg ctatcgctgc gtggcgtctg 1260
tgcccagcat acccggcctg aaccgcacac agctggtcaa cgtggccatt tttggccccc 1320
cttggatggc attcaaggag aggaaggtgt gggtgaaaga gaatatggtg ttgaatctgt 1380
cttgtgaagc gtcagggcac ccccggccca ccatctcctg gaacgtcaac ggcacggcaa 1440
gtgaacaaga ccaagatcca cagcgagtcc tgagcaccct gaatgtcctc gtgaccccgg 1500
agctgttgga gacaggtgtt gaatgcacgg cctccaacga cctgggcaaa aacaccagca 1560
tcctcttcct ggagctggtc aatttaacca ccctcacacc agactccaac acaaccactg 1620
gcctcagcac ttccactgcc agtcctcata ccagagccaa cagcacctcc acagagagaa 1680
agctgccgga gccggagagc cggggcgtgg tcatcgtggc tgtgattgtg tgcatcctgg 1740
tcctggcggt gctgggcgct gtcctctatt tcctctataa gaagggcaag ctgccgtgca 1800
ggcgctcagg gaagcaggag atcacgctac ccccgtctcg taagagcgaa cttgtagttg 1860
aagttaagtc agataagctc ccagaagaga tgggcctcct gcagggcagc agcggtgaca 1920
agagggctcc gggagaccag ggagagaaat acatcgatct gaggcattag ccccgaatca 1980
cttcagctcc cttccctgcc tggaccattc ccagctccct gctcactctt ctctcagcca 2040
aagcctccaa agggactaga gagaagcctc ctgctcccct cgcctgcaca ccccctttca 2100
gagggccact gggttaggac ctgaggacct cacttggccc tgcaaggccc gcttttcagg 2160
gaccagtcca ccaccatctc ctccacgttg agtgaagctc atcccaagca aggagcccca 2220
gtctcccgag cgggtaggag agtttcttgc agaacgtgtt ttttctttac acacattatg 2280
gctgtaaata cctggctcct gccagcagct gagctgggta gcctctctga gctggtttcc 2340
tgccccaaag gctggcttcc accatccagg tgcaccactg aagtgaggac acaccggagc 2400
caggcgcctg ctcatgttga agtgcgctgt tcacacccgc tccggagagc accccagcag 2460
catccagaag cagctgcagt gttgctgcca ccaccctcct gtctgcctct tcaaagtctc 2520
ctgtgacatt ttttctttgg tcagaagcca ggaactggtg tcattcctta aaagatacgt 2580
gccggggcca ggtgtggtgg ctcacgcctg taatcccagc actttgggag gccgaggcgg 2640
gcggatcaca aagtcaggac gagaccatcc tggctaacac ggtgaaaccc tgtctctact 2700
aaaaatacaa aaaaaaatta gctaggcgta gtggttggca cctatagtcc cagctactcg 2760
gaaggctgaa gcaggagaat ggtatgaatc caggaggtgg agcttgcagt gagccgagac 2820
cgtgccactg cactccagcc tgggcaacac agcgagactc cgtctcgagg aaaaaaaaag 2880
aaaagatacg tgcctgcggt gaggaagctg ggcgctgttt tcgagttcag gtgaattagc 2940
ctcaatcccc cgtgttcact tggctcccat agccctcttg atggatcacg taaaactgaa 3000
aggcagcggg gagcagacaa agatgaggtc tacactgtcc ttcatgggga ttaaagctat 3060
ggttatatta gcaccaaact tctacaaacc aagctcaggg ccccaaccct agaagggccc 3120

aaatgagaga atggtactta gggatggaaa acgggcctgg ctagagcttc gggtgtgtgt 3180
gtctgtctgt gtgtatgcat acatatgtgt gtatatatgg ttttgtcagg tgtgtaaatt 3240
tgcaaattgt ttcctttata tatgtatgta tatatatata tgaaaatata tatatatatg 3300
aaaaataaag cttaattgtc ccagaaatca ta 3332 [?]
[?]agcurymkswbdhvnm cca[?]RNA[?] [?] [?]€; [?]NCBI/NM_006500
2009-12-07[?]1[?]3332 [?] €; 4[?] [?]€[?] [?]V3.3[?]FN1[?]homo
sapiensÿ,)gcccgcgccg gctgtgctgc acagggggag gagagggaac cccaggcgcg agcgggaaga
60
ggggacctgc agccacaact tctctggtcc tctgcatccc ttctgtccct ccacccgtcc 120
ccttccccac cctctggccc ccaccttctt ggaggcgaca acccccggga ggcattagaa 180
gggatttttc ccgcaggttg cgaagggaag caaacttggt ggcaacttgc ctcccggtgc 240
gggcgtctct cccccaccgt ctcaacatgc ttaggggtcc ggggcccggg ctgctgctgc 300
tggccgtcca gtgcctgggg acagcggtgc cctccacggg agcctcgaag agcaagaggc 360
aggctcagca aatggttcag ccccagtccc cggtggctgt cagtcaaagc aagcccggtt 420
gttatgacaa tggaaaacac tatcagataa atcaacagtg ggagcggacc tacctaggca 480
atgcgttggt ttgtacttgt tatggaggaa gccgaggttt taactgcgag agtaaacctg 540
aagctgaaga gacttgcttt gacaagtaca ctgggaacac ttaccgagtg ggtgacactt 600
atgagcgtcc taaagactcc atgatctggg actgtacctg catcggggct gggcgaggga 660
gaataagctg taccatcgca aaccgctgcc atgaaggggg tcagtcctac aagattggtg 720
acacctggag gagaccacat gagactggtg gttacatgtt agagtgtgtg tgtcttggta 780
atggaaaagg agaatggacc tgcaagccca tagctgagaa gtgttttgat catgctgctg 840
ggacttccta tgtggtcgga gaaacgtggg agaagcccta ccaaggctgg atgatggtag 900
attgtacttg cctgggagaa ggcagcggac gcatcacttg cacttctaga aatagatgca 960
acgatcagga cacaaggaca tcctatagaa ttggagacac ctggagcaag aaggataatc 1020
gaggaaacct gctccagtgc atctgcacag gcaacggccg aggagagtgg aagtgtgaga 1080
ggcacacctc tgtgcagacc acatcgagcg gatctggccc cttcaccgat gttcgtgcag 1140
ctgtttacca accgcagcct cacccccagc ctcctcccta tggccactgt gtcacagaca 1200
gtggtgtggt ctactctgtg gggatgcagt ggctgaagac acaaggaaat aagcaaatgc 1260
tttgcacgtg cctgggcaac ggagtcagct gccaagagac agctgtaacc cagacttacg 1320
gtggcaactc aaatggagag ccatgtgtct taccattcac ctacaatggc aggacgttct 1380
actcctgcac cacagaaggg cgacaggacg gacatctttg gtgcagcaca acttcgaatt 1440
atgagcagga ccagaaatac tctttctgca cagaccacac tgttttggtt cagactcgag 1500
gaggaaattc caatggtgcc ttgtgccact tccccttcct atacaacaac cacaattaca 1560
ctgattgcac ttctgagggc agaagagaca acatgaagtg gtgtgggacc acacagaact 1620
atgatgccga ccagaagttt gggttctgcc ccatggctgc ccacgaggaa atctgcacaa 1680
ccaatgaagg ggtcatgtac cgcattggag atcagtggga taagcagcat gacatgggtc 1740
acatgatgag gtgcacgtgt gttgggaatg gtcgtgggga atggacatgc attgcctact 1800
cgcagcttcg agatcagtgc attgttgatg acatcactta caatgtgaac gacacattcc 1860
acaagcgtca tgaagagggg cacatgctga actgtacatg cttcggtcag ggtcggggca 1920
ggtggaagtg tgatcccgtc gaccaatgcc aggattcaga gactgggacg ttttatcaaa 1980
ttggagattc atgggagaag tatgtgcatg gtgtcagata ccagtgctac tgctatggcc 2040
gtggcattgg ggagtggcat tgccaacctt tacagaccta tccaagctca agtggtcctg 2100
tcgaagtatt tatcactgag actccgagtc agcccaactc ccaccccatc cagtggaatg 2160
caccacagcc atctcacatt tccaagtaca ttctcaggtg gagacctaaa aattctgtag 2220
gccgttggaa ggaagctacc ataccaggcc acttaaactc ctacaccatc aaaggcctga 2280
agcctggtgt ggtatacgag ggccagctca tcagcatcca gcagtacggc caccaagaag 2340
tgactcgctt tgacttcacc accaccagca ccagcacacc tgtgaccagc aacaccgtga 2400
caggagagac gactcccttt tctcctcttg tggccacttc tgaatctgtg accgaaatca 2460
cagccagtag ctttgtggtc tcctgggtct cagcttccga caccgtgtcg ggattccggg 2520
tggaatatga gctgagtgag gagggagatg agccacagta cctggatctt ccaagcacag 2580
ccacttctgt gaacatccct gacctgcttc ctggccgaaa atacattgta aatgtctatc 2640
agatatctga ggatggggag cagagtttga tcctgtctac ttcacaaaca acagcgcctg 2700
atgcccctcc tgacccgact gtggaccaag ttgatgacac ctcaattgtt gttcgctgga 2760
gcagacccca ggctcccatc acagggtaca gaatagtcta ttcgccatca gtagaaggta 2820
gcagcacaga actcaacctt cctgaaactg caaactccgt caccctcagt gacttgcaac 2880
ctggtgttca gtataacatc actatctatg ctgtggaaga aaatcaagaa agtacacctg 2940
ttgtcattca acaagaaacc actggcaccc cacgctcaga tacagtgccc tctcccaggg 3000
acctgcagtt tgtggaagtg acagacgtga aggtcaccat catgtggaca ccgcctgaga 3060
gtgcagtgac cggctaccgt gtggatgtga tccccgtcaa cctgcctggc gagcacgggc 3120
agaggctgcc catcagcagg aacacctttg cagaagtcac cgggctgtcc cctggggtca 3180
cctattactt caaagtcttt gcagtgagcc atgggaggga gagcaagcct ctgactgctc 3240
aacagacaac caaactggat gctcccacta acctccagtt tgtcaatgaa actgattcta 3300
ctgtcctggt gagatggact ccacctcggg cccagataac aggataccga ctgaccgtgg 3360
gccttacccg aagaggacag cccaggcagt acaatgtggg tccctctgtc tccaagtacc 3420

cactgaggaa tctgcagcct gcatctgagt acaccgtatc cctcgtggcc ataaagggca 3480
accaagagag ccccaaagcc actggagtct ttaccacact gcagcctggg agctctattc 3540
caccttacaa caccgaggtg actgagacca ccattgtgat cacatggacg cctgctccaa 3600
gaattggttt taagctgggt gtacgaccaa gccagggagg agaggcacca cgagaagtga 3660
cttcagactc aggaagcatc gttgtgtccg gcttgactcc aggagtagaa tacgtctaca 3720
ccatccaagt cctgagagat ggacaggaaa gagatgcgcc aattgtaaac aaagtggtga 3780
caccattgtc tccaccaaca aacttgcatc tggaggcaaa ccctgacact ggagtgctca 3840
cagtctcctg ggagaggagc accaccccag acattactgg ttatagaatt accacaaccc 3900
ctacaaacgg ccagcaggga aattctttgg aagaagtggt ccatgctgat cagagctcct 3960
gcacttttga taacctgagt cccggcctgg agtacaatgt cagtgtttac actgtcaagg 4020
atgacaagga aagtgtccct atctctgata ccatcatccc agctgttcct cctcccactg 4080
acctgcgatt caccaacatt ggtccagaca ccatgcgtgt cacctgggct ccacccccat 4140
ccattgattt aaccaacttc ctggtgcgtt actcacctgt gaaaaatgag gaagatgttg 4200
cagagttgtc aatttctcct tcagacaatg cagtggtctt aacaaatctc ctgcctggta 4260
cagaatatgt agtgagtgtc tccagtgtct acgaacaaca tgagagcaca cctcttagag 4320
gaagacagaa aacaggtctt gattccccaa ctggcattga cttttctgat attactgcca 4380
actcttttac tgtgcactgg attgctcctc gagccaccat cactggctac aggatccgcc 4440
atcatcccga gcacttcagt gggagacctc gagaagatcg ggtgccccac tctcggaatt 4500
ccatcaccct caccaacctc actccaggca cagagtatgt ggtcagcatc gttgctctta 4560
atggcagaga ggaaagtccc ttattgattg gccaacaatc aacagtttct gatgttccga 4620
gggacctgga agttgttgct gcgaccccca ccagcctact gatcagctgg gatgctcctg 4680
ctgtcacagt gagatattac aggatcactt acggagagac aggaggaaat agccctgtcc 4740
aggagttcac tgtgcctggg agcaagtcta cagctaccat cagcggcctt aaacctggag 4800
ttgattatac catcactgtg tatgctgtca ctggccgtgg agacagcccc gcaagcagca 4860
agccaatttc cattaattac cgaacagaaa ttgacaaacc atcccagatg caagtgaccg 4920
atgttcagga caacagcatt agtgtcaagt ggctgccttc aagttcccct gttactggtt 4980
acagagtaac caccactccc aaaaatggac caggaccaac aaaaactaaa actgcaggtc 5040
cagatcaaac agaaatgact attgaaggct tgcagcccac agtggagtat gtggttagtg 5100
tctatgctca gaatccaagc ggagagagtc agcctctggt tcagactgca gtaaccacta 5160
ttcctgcacc aactgacctg aagttcactc aggtcacacc cacaagcctg agcgcccagt 5220
ggacaccacc caatgttcag ctcactggat atcgagtgcg ggtgaccccc aaggagaaga 5280
ccggaccaat gaaagaaatc aaccttgctc ctgacagctc atccgtggtt gtatcaggac 5340
ttatggtggc caccaaatat gaagtgagtg tctatgctct taaggacact ttgacaagca 5400
gaccagctca gggagttgtc accactctgg agaatgtcag cccaccaaga agggctcgtg 5460
tgacagatgc tactgagacc accatcacca ttagctggag aaccaagact gagacgatca 5520
ctggcttcca agttgatgcc gttccagcca atggccagac tccaatccag agaaccatca 5580
agccagatgt cagaagctac accatcacag gtttacaacc aggcactgac tacaagatct 5640
acctgtacac cttgaatgac aatgctcgga gctcccctgt ggtcatcgac gcctccactg 5700
ccattgatgc accatccaac ctgcgtttcc tggccaccac acccaattcc ttgctggtat 5760
catggcagcc gccacgtgcc aggattaccg gctacatcat caagtatgag aagcctgggt 5820
ctcctcccag agaagtggtc cctcggcccc gccctggtgt cacagaggct actattactg 5880
gcctggaacc gggaaccgaa tatacaattt atgtcattgc cctgaagaat aatcagaaga 5940
gcgagcccct gattggaagg aaaaagacag acgagcttcc ccaactggta acccttccac 6000
accccaatct tcatggacca gagatcttgg atgttccttc cacagttcaa aagacccctt 6060
tcgtcaccca ccctgggtat gacactggaa atggtattca gcttcctggc acttctggtc 6120
agcaacccag tgttgggcaa caaatgatct ttgaggaaca tggttttagg cggaccacac 6180
cgcccacaac ggccaccccc ataaggcata ggccaagacc atacccgccg aatgtaggtg 6240
aggaaatcca aattggtcac atccccaggg aagatgtaga ctatcacctg tacccacacg 6300
gtccgggact caatccaaat gcctctacag gacaagaagc tctctctcag acaaccatct 6360
catgggcccc attccaggac acttctgagt acatcatttc atgtcatcct gttggcactg 6420
atgaagaacc cttacagttc agggttcctg gaacttctac cagtgccact ctgacaggcc 6480
tcaccagagg tgccacctac aacatcatag tggaggcact gaaagaccag cagaggcata 6540
aggttcggga agaggttgtt accgtgggca actctgtcaa cgaaggcttg aaccaaccta 6600
cggatgactc gtgctttgac ccctacacag tttcccatta tgccgttgga gatgagtggg 6660
aacgaatgtc tgaatcaggc tttaaactgt tgtgccagtg cttaggcttt ggaagtggtc 6720
atttcagatg tgattcatct agatggtgcc atgacaatgg tgtgaactac aagattggag 6780
agaagtggga ccgtcaggga gaaaatggcc agatgatgag ctgcacatgt cttgggaacg 6840
gaaaaggaga attcaagtgt gaccctcatg aggcaacgtg ttatgatgat gggaagacat 6900
accacgtagg agaacagtgg cagaaggaat atctcggtgc catttgctcc tgcacatgct 6960
ttggaggcca gcggggctgg cgctgtgaca actgccgcag acctgggggt gaacccagtc 7020
ccgaaggcac tactggccag tcctacaacc agtattctca gagataccat cagagaacaa 7080
acactaatgt taattgccca attgagtgct tcatgccttt agatgtacag gctgacagag 7140
aagattcccg agagtaaatc atctttccaa tccagaggaa caagcatgtc tctctgccaa 7200
gatccatcta aactggagtg atgttagcag acccagctta gagttcttct ttctttctta 7260

agccctttgc tctggaggaa gttctccagc ttcagctcaa ctcacagctt ctccaagcat 7320
caccctggga gtttcctgag ggttttctca taaatgaggg ctgcacattg cctgttctgc 7380
ttcgaagtat tcaataccgc tcagtatttt aaatgaagtg attctaagat ttggtttggg 7440
atcaatagga aagcatatgc agccaaccaa gatgcaaatg ttttgaaatg atatgaccaa 7500
aattttaagt aggaaagtca cccaaacact tctgctttca cttaagtgtc tggcccgcaa 7560
tactgtagga acaagcatga tcttgttact gtgatatttt aaatatccac agtactcact 7620
ttttccaaat gatcctagta attgcctaga aatatctttc tcttacctgt tatttatcaa 7680
tttttcccag tatttttata cggaaaaaat tgtattgaaa acacttagta tgcagttgat 7740
aagaggaatt tggtataatt atggtgggtg attatttttt atactgtatg tgccaaagct 7800
ttactactgt ggaaagacaa ctgttttaat aaaagattta cattccacaa cttgaagttc 7860
atctatttga tataagacac cttcggggga aataattcct gtgaatattc tttttcaatt 7920
cagcaaacat ttgaaaatct atgatgtgca agtctaattg ttgatttcag tacaagattt 7980
tctaaatcag ttgctacaaa aactgattgg tttttgtcac ttcatctctt cactaatgga 8040
gatagcttta cactttctgc tttaatagat ttaagtggac cccaatattt attaaaattg 8100
ctagtttacc gttcagaagt ataatagaaa taatctttag ttgctctttt ctaaccattg 8160
taattcttcc cttcttccct ccacctttcc ttcattgaat aaacctctgt tcaaagagat 8220
tgcctgcaag ggaaataaaa atgactaaga tattaaaaaa aaaaaaaaaa aa 8272
dcÉ [?] [?]agcurymkswbdhvnmgcag[?]RNA[?] [?] [?]€> [?]NCBI/NM_212476
2009-12-06[?]1[?]8272 [?] €> <tR [?]€[?] [?]V3.3"LOC653879
similar to complement C3[?]homo sapiensÿ6[?]cttaacaaat ttctgaccac agccaaagat aagaaccgct
gggaggaccc tggtaagcag 60
ctctacaacg tggaggccac atcctatgcc ctcttggccc tactgcagct aaaagacttt 120
gactttgtgc ctcccgtcgt gcgttggctc aatgaacaga gatactacgg tggtggctat 180
ggctctaccc aggccacctt catggtgttc caagccttgg ctcaatacca aaaggacgcc 240
cctgaccacc aggaactgaa ccttgatgtg tccctccaac tgcccagccg cagctccaag 300
atcacccacc gtatccactg ggaatctgcc agcctcctgc gatcagaaga gaccaaggaa 360
aatgagggtt tcacagtcac agctgaagga aaaggccaag gcaccttgtc ggtggtgaca 420
atgtaccatg ctaaggccaa agatcaactc acctgtaata aattcgacct caaggtcacc 480
ataaaaccag caccggaaac agaaaagagg cctcaggatg ccaagaacac tatgatcctt 540
gagatctgta ccaggtaccg gggagaccag gatgccacta tgtctatatt ggacatatcc 600
atgatgactg gctttgctcc agacacagat gacctgaagc agctggccaa tggtgttgac 660
agatacatct ccaagtatga gctggacaaa gccttctccg ataggaacac cctcatcatc 720
tacctggaca aggtctcaca ctctgaggat gactgtctag ctttcaaagt tcaccaatac 780
tttaatgtag agcttatcca gcctggagca gtcaaggtct acgcctatta caacctggag 840
gaaagctgta cccggttcta ccatccggaa aaggaggatg gaaagctgaa caagctctgc 900
cgtgatgaac tgtgccgctg tgctgaggag aattgcttca tacaaaagtc ggatgacaag 960
gtcaccctgg aagaacggct ggacaaggcc tgtgagccag gagtggacta tgtgtacaag 1020
acccgactgg tcaaggttca gctgtccaat gactttgacg agtacatcat ggccattgag 1080
cagaccatca agtcaggctc ggatgaggtg caggttggac agcagcgcac gttcatcagc 1140
cccatcaagt gcagagaagc cctgaagctg gaggagaaga aacactacct catgtggggt 1200
ctctcctccg atttctgggg agagaagccc aacctcagct acatcatcgg gaaggacact 1260
tgggtggagc actggcctga ggaggacgaa tgccaagacg aagagaacca gaaacaatgc 1320
caggacctcg gcgccttcac cgagagcatg gttgtctttg ggtgccccaa ctgaccacac 1380
ccccattcca tgaacctaca gaga 1404ta a[?]
[?]agcurymkswbdhvnmggct[?]RNA[?] [?] [?]€A [?]NCBI/XP_001724196
2009-06-10[?]1[?]1404 [?] €A HCTD [?] ÿÿ[?]
COrgApplicant[?] [?]15385 NE 90th Street[?]Redmond
Washington[?]USA[?]98052[?]425-2844390[?]425-2844396 [?]Onconome Inc[?] ÿÿ[?] [?] CIndivApplicant[?]
[?]114 N 200th Street Shoreline
Washington[?]USA[?]98133[?]425-2844390 x321[?]425-2844396[?]eaw@onconome.com[?]Eva [?]Aw

ÿÿ□ □ CProject ÿÿ□ □ CProjectData□ ICompositions and Methods for Diagnosis and Prognosis of Colorectal Cancer□PCT_12112009
____-__-__ □ ÿÿ□ □ CProtein1Sequence□ □V3.3□ORM1□homo sapiensÿ2□MALSWVLTVL
SLLPLLEAQI PLCANLVPVP ITNATLDQIT GKWFYIASAF RNEEYNKSVQ 60
EIQATFFYFT PNKTEDTIFL REYQTRQDQC IYNTTYLNVQ RENGTISRYV GGQEHFAHLL 120
ILRDTKTYML AFDVNDEKNW GLSVYADKPE TTKEQLGEFY EALDCLRIPK SDVVYTDWKK 180
DKCEPLEKQH EKERKQEEGE S 201□ □
□ARNDBCQEZGHILKMFPSTWYVX□ □PRT□ □ ÿÿ□ □ CPubDatabase□ □Swiss-Prot/P02763
1988-01-01□1□201 □ ÿÿ□ □ CCommentFeature□ 4□ □€□ □V3.3
C2 complement□homo sapiensÿç□MGPLMVLFCL LFLYPGLADS APSCPQNVNI SGGTFTLSHG WAPGSLLTYS
CPQGLYPSPA 60
SRLCKSSGQW QTPGATRSLS KAVCKPVRCP APVSFENGIY TPRLGSYPVG GNVSFECEDG 120
FILRGSPVRQ CRPNGMWDGE TAVCDNGAGH CPNPGISLGA VRTGFRFGHG DKVRYRCSSN 180
LVLTGSSERE CQGNGVWSGT EPICRQPYSY DFPEDVAPAL GTSFSHMLGA TNPTQKTKES 240
LGRKIQIQRS GHLNLYLLLD CSQSVSENDF LIFKESASLM VDRIFSFEIN VSVAIITFAS 300
EPKVLMSVLN DNSRDMTEVI SSLENANYKD HENGTGTNTY AALNSVYLMM NNQMRLLGME 360
TMAWQEIRHA IILLTDGKSN MGGSPKTAVD HIREILNINQ KRNDYLDIYA IGVGKLDVDW 420
RELNELGSKK DGERHAFILQ DTKALHQVFE HMLDVSKLTD TICGVGNMSA NASDQERTPW 480
HVTIKPKSQE TCRGALISDQ WVLTAAHCFR DGNDHSLWRV NVGDPKSQWG KEFLIEKAVI 540
SPGFDVFAKK NQGILEFYGD DIALLKLAQK VKMSTHARPI CLPCTMEANL ALRRPQGSTC 600
RDHENELLNK QSVPAHFVAL NGSKLNINLK MGVEWTSCAE VVSQEKTMFP NLTDVREVVT 660
DQFLCSGTQE DESPCKGESG GAVFLERRFR FFQVGLVSWG LYNPCLGSAD KNSRKRAPRS 720
KVPPPRDFHI NLFRMQPWLR QHLGDVLNFL PL 752 □ □
□ARNDBCQEZGHILKMFPSTWYVX□ □PRT□ □ □€□ □Swiss-Prot/P06681
1998-12-15□1□752 □ €□ 4□ □€□ □V3.3□C-Reactive
Protein□homo sapiensÿ2□MEKLLCFLVL TSLSHAFGQT DMSRKAFVFP KESDTSYVSL KAPLTKPLKA
FTVCLHFYTE 60
LSSTRGYSIF SYATKRQDNE ILIFWSKDIG YSFTVGGSEI LFEVPEVTVA PVHICTSWES 120
ASGIVEFWVD GKPRVRKSLK KGYTVGAEAS IILGQEQDSF GGNFEGSQSL VGDIGNVNMW 180
DFVLSPDEIN TIYLGGPFSP NVLNWRALKY EVQGEVFTKP QLWP 224LL S□
□ARNDBCQEZGHILKMFPSTWYVXSFTL□PRT□ □ □€□ □Swiss-Prot/P02741
1988-01-01□1□224 □ €□ □€□ □V3.3□CFHR1□home
sapiensÿÌ□MWLLVSVILI SRISSVGGEA TFCDFPKINH GILYDEEKYK PFSQVPTGEV FYYSCEYNFV
60
SPSKSFWTRI TCTEEGWSPT PKCLRLCFFP FVENGHSESS GQTHLEGDTV QIICNTGYRL 120
QNNENNISCV ERGWSTPPKC RSTDTSCVNP PTVQNAHILS RQMSKYPSGE RVRYECRSPY 180
EMFGDEEVMC LNGNWTEPPQ CKDSTGKCGP PPPIDNGDIT SFPLSVYAPA SSVEYQCQNL 240
YQLEGNKRIT CRNGQWSEPP KCLHPCVISR EIMENYNIAL RWTAKQKLYL RTGESAEFVC 300
KRGYRLSSRS HTLRTTCWDG KLEYPTCAKR 330KA F□
□ARNDBCQEZGHILKMFPSTWYVXKDIG□PRT□ □ □€□ □Swiss-Prot/Q03591
2008-12-16□1□330 □ €□ □€□ □V3.3□SERPINA3□homo
sapiensÿf□MERMLPLLAL GLLAAGFCPA VLCHPNSPLD EENLTQENQD RGTHVDLGLA SANVDFAFSL
60
YKQLVLKAPD KNVIFSPLSI STALAFLSLG AHNTTLTEIL KGLKFNLTET SEAEIHQSFQ 120
HLLRTLNQSS DELQLSMGNA MFVKEQLSLL DRFTEDAKRL YGSEAFATDF QDSAAAKKLI 180
NDYVKNGTRG KITDLIKDLD SQTMMVLVNY IFFKAKWEMP FDPQDTHQSR FYLSKKKWVM 240
VPMMSLHHLT IPYFRDEELS CTVVELKYTG NASALFILPD QDKMEEVEAM LLPETLKRWR 300
DSLEFREIGE LYLPKFSISR DYNLNDILLQ LGIEEAFTSK ADLSGITGAR NLAVSQVVHK 360
AVLDVFEEGT EASAATAVKI TLLSALVETR TIVRFNRPFL MIIVPTDTQN IFFMSKVTNP 420
KQA 423VFTL□
□ARNDBCQEZGHILKMFPSTWYVX SYC□PRT□ □ □€□ □Swiss-Prot/P01011
1991-08-01□1□423 □ €□ LFRM □€□ □V3.3□HABP2□homo
sapiensÿ □MFARMSDLHV LLLMALVGKT ACGFSLMSLL ESLDPDWTPD QYDYSYEDYN QEENTSSTLT
60
HAENPDWYYT EDQADPCQPN PCEHGGDCLV HGSTFTCSCL APFSGNKCQK VQNTCKDNPC 120
GRGQCLITQS PPYYRCVCKH PYTGPSCSQV VPVCRPNPCQ NGATCSRHKR RSKFTCACPD 180
QFKGKFCEIG SDDCYVGDGY SYRGKMNRTV NQHACLYWNS HLLLQENYNM FMEDAETHGI 240
GEHNFCRNPD ADEKPWCFIK VTNDKVKWEY CDVSACSAQD VAYPEESPTE PSTKLPGFDS 300
CGKTEIAERK IKRIYGGFKS TAGKHPWQAS LQSSLPLTIS MPQGHFCGGA LIHPCWVLTA 360
AHCTDIKTRH LKVVLGDQDL KKEEFHEQSF RVEKIFKYSH YNERDEIPHN DIALLKLKPV 420
DGHCALESKY VKTVCLPDGS FPSGSECHIS GWGVTETGKG SRQLLDAKVK LIANTLCNSR 480
QLYDHMIDDS MICAGNLQKP GQDTCQGDSG GPLTCEKDGT YYVYGIVSWG LECGKRPGVY 540
TQVTKFLNWI KATIKSESGF 560|□x □
□ARNDBCQEZGHILKMFPSTWYVXQDIP□PRT□ □ □€□ □Swiss-Prot/Q14520

1996-11-01[?]1[?]560 [?] €[?] [?]€[?] [?]V3.3[?]CNDP1[?]homo
sapiensÿ³[?]MDPKLGRMAA SLLAVLLLLL ERGMFSSPSP PPALLEKVFQ YIDLHQDEFV QTLKEWVAIE
60
SDSVQPVPRF RQELFRMMAV AADTLQRLGA RVASVDMGPQ QLPDGQSLPI PPVILAELGS 120
DPTKGTVCFY GHLDVQPADR GDGWLTDPYV LTEVDGKLYG RGATDNKGPV LAWINAVSAF 180
RALEQDLPVN IKFIIEGMEE AGSVALEELV EKEKDRFFSG VDYIVISDNL WISQRKPAIT 240
YGTRGNSYFM VEVKCRDQDF HSGTFGGILH EPMADLVALL GSLVDSSGHI LVPGIYDEVV 300
PLTEEEINTY KAIHLDLEEY RNSSRVEKFL FDTKEEILMH LWRYPSLSIH GIEGAFDEPG 360
TKTVIPGRVI GKFSIRLVPH MNVSAVEKQV TRHLEDVFSK RNSSNKMVVS MTLGLHPWIA 420
NIDDTQYLAA KRAIRTVFGT EPDMIRDGST IPIAKMFQEI VHKSVVLIPL GAVDDGEHSQ 480
NEKINRWNYI EGTKLFAAFF LEMAQLH 507$[?]É [?]
[?]ARNDBCQEZGHILKMFPSTWYVX[?] [?]PRT[?] [?] [?]€[?] [?]Swiss-Prot/Q96KN2
2006-07-25[?]1[?]507 [?] €[?] [?]€[?] [?]V3.3[?]CFHR2[?]homo
sapiensÿ[?][?]MWLLVSVILI SRISSVGGEA MFCDFPKINH GILYDEEKYK PFSQVPTGEV FYYSCEYNFV
60
SPSKSFWTRI TCAEEGWSPT PKCLRLCFFP FVENGHSESS GQTHLEGDTV QIICNTGYRL 120
QNNENNISCV ERGWSTPPKC RSTISAEKCG PPPPIDNGDI TSFLLSVYAP GSSVEYQCQN 180
LYQLEGNNQI TCRNGQWSEP PKCLDPCVIS QEIMEKYNIK LKWTNQQKLY SRTGDIVEFV 240
CKSGYHPTKS HSFRAMCQNG KLVYPSCEEK 270 [?]
[?]ARNDBCQEZGHILKMFPSTWYVXV SV[?]PRT[?] [?] [?]€[?] [?]Swiss-Prot/P36980
1994-06-01[?]1[?]270 [?] €[?] lyti [?]€[?] [?]V3.3[?]SAA[?]homo
sapiensåMKLLTGLVFC SLVLGVSSRS FFSFLGEAFD GARDMWRAYS DMREANYIGS DKYFHARGNY 60
DAAKRGPGGV WAAEAISDAR ENIQRFFGHG AEDSLADQAA NEWGRSGKDP NHFRPAGLPE 120
KY 122 [?]
[?]ARNDBCQEZGHILKMFPSTWYVXESS [?]PRT[?] [?] [?]€ [?]Swiss-Prot/P02735
1990-08-01[?]1[?]122 [?] € [?]€[?] [?]V3.3[?]PROZ[?]homo
sapiensÿ[?][?]MAGCVPLLQG LVLVLALHRV .EPSVFLPASK ANDVLVRWKR AGSYLLEELF EGNLEKECYE
60
EICVYEEARE VFENEVVTDE FWRRYKGGSP CISQPCLHNG SCQDSIWGYT CTCSPGYEGS 120
NCELAKNECH PERTDGCQHF CLPGQESYTC SCAQGYRLGE DHKQCVPHDQ CACGVLTSEK 180
RAPDLQDLPW QVKLTNSEGK DFCGGVIIRE NFVLTTAKCS LLHRNITVKT YFNRTSQDPL 240
MIKITHVHVH MRYDADAGEN DLSLLELEWP IQCPGAGLPV CTPEKDFAEH LLIPRTRGLL 300
SGWARNGTDL GNSLTTRPVT LVEGEECGQV LNVTVTTRTY CERSSVAAMH WMDGSVVTRE 360
HRGSWFLTGV LGSQPVGGQA HMVLVTKVSR YSLWFKQIMN 400GS D[?]
[?]ARNDBCQEZGHILKMFPSTWYVXFGHG[?]PRT[?] [?] [?]€# [?]Swiss-Prot/P22891
1991-11-01[?]1[?]400 [?] €# EK D [?]€[?] [?]V3.3[?]PON3[?]homo
sapiensÿÎ[?]MGKLVALVLL GVGLSLVGEM FLAFRERVNA SREVEPVEPE NCHLIEELES GSEDIDILPS
60
GLAFISSGLK YPGMPNFAPD EPGKIFLMDL NEQNPRAQAL EISGGFDKEL FNPHGISIFI 120
DKDNTVYLYV VNHPHMKSTV EIFKFEEQQR SLVYLKTIKH ELLKSVNDIV VLGPEQFYAT 180
RDHYFTNSLL SFFEMILDLR WTYVLFYSPR EVKVVAKGFC SANGITVSAD QKYVYVADVA 240
AKNIHIMEKH DNWDLTQLKV IQLGTLVDNL TVDPATGDIL AGCHPNPMKL LNYNPEDPPG 300
SEVLRIQNVL SEKPRVSTVY ANNGSVLQGT SVASVYHGKI LIGTVFHKTL YCEL 354
[?] [?]ARNDBCQEZGHILKMFPSTWYVXHNG [?]PRT[?] [?] [?]€& [?]Swiss-Prot/Q15166
2004-07-19[?]1[?]354 [?] €& VVTR [?]€[?] [?]V3.3[?]RARRES2[?]homo
sapiensåMRRLLIPLAL WLGAVGVGVA ELTEAQRRGL QVALEEFHKH PPVQWAFQET SVESAVDTPF 60
PAGIFVRLEF KLQQTSCRKR DWKKPECKVR PNGRKRKCLA CIKLGSEDKV LGRLVHCPIE 120
TQVLREAEEH QETQCLRVQR AGEDPHSFYF PGQFAFSKAL PRS 163DøÇ [?]
[?]ARNDBCQEZGHILKMFPSTWYVXLMDL[?]PRT[?] [?] [?]€) [?]Swiss-Prot/Q99969
1997-05-01[?]1[?]163 [?] €) TL Y [?]€[?] [?]V3.3[?]GGH[?]homo
sapiensÿÌ[?]MASPGCLLCV LGLLLCGAAS LELSRPHGDT AKKPIIGILM QKCRNKVMKN YGRYYIAASY
60
VKYLESAGAR VVPVRLDLTE KDYEILFKSI NGILFPGGSV DLRRSDYAKV AKIFYNLSIQ 120
SFDDGDYFPV WGTCLGFEEL SLLISGECLL TATDTVDVAM PLNFTGGQLH SRMFQNFPTE 180
LLLSLAVEPL TANFHKWSLS VKNFTMNEKL KKFFNVLTTN TDGKIEFIST MEGYKYPVYG 240
VQWHPEKAPY EWKNLDGISH APNAVKTAFY LAEFFVNEAR KNNHHFKSES EEEKALIYQF 300
SPIYTGNISS FQQCYIFD 318ET S[?]
[?]ARNDBCQEZGHILKMFPSTWYVXCKVR[?]PRT[?] [?] [?]€, [?]Swiss-Prot/Q92820
1998-08-01[?]1[?]318 [?] €, [?]€[?] [?]V3.3[?]PRG4[?]homo
sapiensÿ6[?]MAWKTLPIYL LLLLLSVFVIQ QVSSQDLSSC AGRCGEGYSR DATCNCDYNC QHYMECCPDF
60
KRVCTAELSC KGRCFESFER GRECDCDAQC KKYDKCCPDY ESFCAEVHNP TSPPSSKKAP 120
PPSGASQTIK STTKRSPKPP NKKKTKKVIE SEEITEEHSV SENQESSSSS SSSSSSSTIR 180
KIKSSKNSAA NRELQKKLKV KDNKKNRTKK KPTPKPPVVD EAGSGLDNGD FKVTTPDTST 240

PCT112112009.txt
TQHNKVSTSP KITTAKPINP RPSLPPNSDT SKETSLTVNK ETTVETKETT TTNKQTSTDG 300
KEKTTSAKET QSIEKTSAKD LAPTSKVLAK PTPKAETTTK GPALTTPKEP TPTTPKEPAS 360
TTPKEPTPTT IKSAPTTPKE PAPTTTKSAP TTPKEPAPTT TKEPAPTTPK EPAPTTTKEP 420
APTTTKSAPT TPKEPAPTTP KKPAPTTPKE PAPTTPKEPT PTTPKEPAPT TKEPAPTTPK 480
EPAPTAPKKP APTTPKEPAP TTPKEPAPTT TKEPSPTTPK EPAPTTTKSA PTTTKEPAPT 540
TTKSAPTTPK EPSPTTTKEP APTTPKEPAP TTPKKPAPTT PKEPAPTTPK EPAPTTTKKP 600
APTTPKEPAP TTPKETAPTT PKKLTPTTPE KLAPTTPEKP APTTPEELAP TTPEEPTPTT 660
PEEPAPTTPK AAAPNTPKEP APTTPKEPAP TTPKEPAPTT PKETAPTTPK GTAPTTLKEP 720
APTTPKKPAP KELAPTTTKE PTSTTCDKPA PTTPKGTAPT TPKEPAPTTP KEPAPTTPKG 780
TAPTTLKEPA PTTPKKPAPK ELAPTTTKGP TSTTSDKPAP TTPKETAPTT PKEPAPTTPK 840
KPAPTTPETP PPTTSEVSTP TTTKEPTTIH KSPDESTPEL SAEPTPKALE NSPKEPGVPT 900
TKTPAATKPE MTTTAKDKTT ERDLRTTPET TTAAPKMTKE TATTTEKTTE SKITATTTQV 960
TSTTTQDTTP FKITTLKTTT LAPKVTTTKK TITTTEIMNK PEETAKPKDR ATNSKATTPK 1020
PQKPTKAPKK PTSTKKPKTM PRVRKPKTTP TPRKMTSTMP ELNPTSRIAE AMLQTTTRPN 1080
QTPNSKLVEV NPKSEDAGGA EGETPHMLLR PHVFMPEVTP DMDYLPRVPN QGIIINPMLS 1140
DETNICNGKP VDGLTTLRNG TLVAFRGHYF WMLSPFSPPS PARRITEVWG IPSPIDTVFT 1200
RCNCEGKTFF FKDSQYWRFT NDIKDAGYPK PIFKGFGGLT GQIVAALSTA KYKNWPESVY 1260
FFKRGGSIQQ YIYKQEPVQK CPGRRPALNY PVYGETTQVR RRRFERAIGP SQTHTIRIQY 1320
SPARLAYQDK GVLHNEVKVS ILWRGLPNVV TSAISLPNIR KPDGYDYYAF SKDQYYNIDV 1380
PSRTARAITT RSGQTLSKVW YNCP 1404„ÇÇ [?]
[?]ARNDBCQEZGHILKMFPSTWYVXFKSI[?]PRT[?] [?] [?]€/ [?]Swiss-Prot/Q92954
2005-12-06[?]1[?]1404 [?] €/ [?]€[?] [?]V3.3[?]MCAM[?]homo
sapiensÿM[?]MGLPRLVCAF LLAACCCCPR VAGVPGEAEQ PAPELVEVEV GSTALLKCGL SQSQGNLSHV
60
DWFSVHKEKR TLIFRVRQGQ GQSEPGEYEQ RLSLQDRGAT LALTQVTPQD ERIFLCQGKR 120
PRSQEYRIQL RVYKAPEEPN IQVNPLGIPV NSKEPEEVAT CVGRNGYPIP QVIWYKNGRP 180
LKEEKNRVHI QSSQTVESSG LYTLQSILKA QLVKEDKDAQ FYCELNYRLP SGNHMKESRE 240
VTVPVFYPTE KVWLEVEPVG MLKEGDRVEI RCLADGNPPP HFSISKQNPS TREAEEETTN 300
DNGVLVLEPA RKEHSGRYEC QGLDLDTMIS LLSEPQELLV NYVSDVRVSP AAPERQEGSS 360
LTLTCEAESS QDLEFQWLRE ETGQVLERGP VLQLHDLKRE AGGGYRCVAS VPSIPGLNRT 420
QLVNVAIFGP PWMAFKERKV WVKENMVLNL SCEASGHPRP TISWNVNGTA SEQDQDPQRV 480
LSTLNVLVTP ELLETGVECT ASNDLGKNTS ILFLELVNLT TLTPDSNTTT GLSTSTASPH 540
TRANSTSTER KLPEPESRGV VIVAVIVCIL VLAVLGAVLY FLYKKGKLPC RRSGKQEITL 600
PPSRKSELVV EVKSDKLPEE MGLLQGSSGD KRAPGDQGEK YIDLRH 646"ÐÇ [?]
[?]ARNDBCQEZGHILKMFPSTWYVXTGFR[?]PRT[?] [?] [?]€2 [?]Swiss-Prot/P43121
2006-01-10[?]1[?]646 [?] €2 [?]€[?] [?]V3.3[?]FN1[?]homo
sapiensÿ[?][?]MLRGPGPGLL LLAVQCLGTA VPSTGASKSK RQAQQMVQPQ SPVAVSQSKP GCYDNGKHYQ
60
INQQWERTYL GNALVCTCYG GSRGFNCESK PEAEETCFDK YTGNTYRVGD TYERPKDSMI 120
WDCTCIGAGR GRISCTIANR CHEGGQSYKI GDTWRRPHET GGYMLECVCL GNGKGEWTCK 180
PIAEKCFDHA AGTSYVVGET WEKPYQGWMM VDCTCLGEGS GRITCTSRNR CNDQDTRTSY 240
RIGDTWSKKD NRGNLLQCIC TGNGRGEWKC ERHTSVQTTS SGSGPFTDVR AAVYQPQPHP 300
QPPPYGHCVT DSGVVYSVGM QWLKTQGNKQ MLCTCLGNGV SCQETAVTQT YGGNSNGEPC 360
VLPFTYNGRT FYSCTTEGRQ DGHLWCSTTS NYEQDQKYSF CTDHTVLVQT QGGNSNGALC 420
HFPFLYNNHN YTDCTSEGRR DNMKWCGTTQ NYDADQKFGF CPMAAHEEIC TTNEGVMYRI 480
GDQWDKQHDM GHMMRCTCVG NGRGEWTCIA YSQLRDQCIV DDITYNVNDT FHKRHEEGHM 540
LNCTCFGQGR GRWKCDPVDQ CQDSETGTFY QIGDSWEKYV HGVRYQCYCY GRGIGEWHCQ 600
PLQTYPSSSG PVEVFITETP SQPNSHPIQW NAPQPSHISK YILRWRPKNS VGRWKEATIP 660
GHLNSYTIKG LKPGVVYEGQ LISIQQYGHQ EVTRFDFTTT STSTPVTSNT VTGETTPFSP 720
LVATSESVTE ITASSFVVSW VSASDTVSGF RVEYELSEEG DEPQYLDLPS TATSVNIPDL 780
LPGRKYIVNV YQISEDGEQS LILSTSQTTA PDAPPDPTVD QVDDTSIVVR WSRPQAPITG 840
YRIVYSPSVE GSSTELNLPE TANSVTLSDL QPGVQYNITI YAVEENQEST PVVIQQETTG 900
TPRSDTVPSP RDLQFVEVTD VKVTIMWTPP ESAVTGYRVD VIPVNLPGEH GQRLPISRNT 960
FAEVTGLSPG VTYYFKVFAV SHGRESKPLT AQQTTKLDAP TNLQFVNETD STVLVRWTPP 1020
RAQITGYRLT VGLTRRGQPR QYNVGPSVSK YPLRNLQPAS EYTVSLVAIK GNQESPKATG 1080
VFTTLQPGSS IPPYNTEVTE TTIVITWTPA PRIGFKLGVR PSQGGEAPRE VTSDSGSIVV 1140
SGLTPGVEYV YTIQVLRDGQ ERDAPIVNKV VTPLSPPTNL HLEANPDTGV LTVSWERSTT 1200
PDITGYRITT TPTNGQQGNS LEEVVHADQS SCTFDNLSPG LEYNVSVYTV KDDKESVPIS 1260
DTIIPAVPPP TDLRFTNIGP DTMRVTWAPP PSIDLTNFLV RYSPVKNEED VAELSISPSD 1320
NAVVLTNLLP GTEYVVSVSS VYEQHESTPL RGRQKTGLDS PTGIDFSDIT ANSFTVHWIA 1380
PRATITGYRI RHHPEHFSGR PREDRVPHSR NSITLTNLTP GTEYVVSIVA LNGREESPLL 1440
IGQQSTVSDV PRDLEVVAAT PTSLLISWDA PAVTVRYYRI TYGETGGNSP VQEFTVPGSK 1500
STATISGLKP GVDYTITVYA VTGRGDSPAS SKPISINYRT EIDKPSQMQV TDVQDNSISV 1560
KWLPSSSPVT GYRVTTTPKN GPGPTKTKTA GPDQTEMTIE GLQPTVEYVV SVYAQNPSGE 1620

SQPLVQTAVT NIDRPKGLAF TDVDVDSIKI AWESPQGQVS RYRVTYSSPE DGIHELFPAP 1680
DGEEDTAELQ GLRPGSEYTV SVVALHDDME SQPLIGTQST AIPAPTDLKF TQVTPTSLSA 1740
QWTPPNVQLT GYRVRVTPKE KTGPMKEINL APDSSSVVVS GLMVATKYEV SVYALKDTLT 1800
SRPAQGVVTT LENVSPPRRA RVTDATETTI TISWRTKTET ITGFQVDAVP ANGQTPIQRT 1860
IKPDVRSYTI TGLQPGTDYK IYLYTLNDNA RSSPVVIDAS TAIDAPSNLR FLATTPNSLL 1920
VSWQPPRARI TGYIIKYEKP GSPPREVVPR PRPGVTEATI TGLEPGTEYT IYVIALKNNQ 1980
KSEPLIGRKK TDELPQLVTL PHPNLHGPEI LDVPSTVQKT PFVTHPGYDT GNGIQLPGTS 2040
GQQPSVGQQM IFEEHGFRRT TPPTTATPIR HRPRPYPPNV GEEIQIGHIP REDVDYHLYP 2100
HGPGLNPNAS TGQEALSQTT ISWAPFQDTS EYIISCHPVG TDEEPLQFRV PGTSTSATLT 2160
GLTRGATYNI IVEALKDQQR HKVREEVVTV GNSVNEGLNQ PTDDSCFDPY TVSHYAVGDE 2220
WERMSESGFK LLCQCLGFGS GHFRCDSSRW CHDNGVNYKI GEKWDRQGEN GQMMSCTCLG 2280
NGKGEFKCDP HEATCYDDGK TYHVGEQWQK EYLGAICSCT CFGGQRGWRC DNCRRPGGEP 2340
SPEGTTGQSY NQYSQRYHQR TNTNVNCPIE CFMPLDVQAD REDSRE 2386GL S[?]
[?]ARNDBCQEZGHILKMFPSTWYVXEYEQ[?]PRT[?] [?] [?]€5 [?]Swiss-Prot/P02751
1997-11-01[?]1[?]2386 [?] €5 66 [?]€[?] [?]V3.3!LOC65389
similar to complement C3[?]homo sapiensÿ[?][?]MVFQALAQYQ KDAPDHQELN LDVSLQLPSR SSKITHRIHW
ESASLLRSEE TKENEGFTVT 60
AEGKGQGTLS VVTMYHAKAK DQLTCNKFDL KVTIKPAPET EKRPQDAKNT MILEICTRYR 120
GDQDATMSIL DISMMTGFAP DTDDLKQLAN GVDRYISKYE LDKAFSDRNT LIIYLDKVSH 180
SEDDCLAFKV HQYFNVELIQ PGAVKVYAYY NLEESCTRFY HPEKEDGKLN KLCRDELCRC 240
AEENCFIQKS DDKVTLEERL DKACEPGVDY VYKTRLVKVQ LSNDFDEYIM AIEQTIKSGS 300
DEVQVGQQRT FISPIKCREA LKLEEKKHYL MWGLSSDFWG EKPNLSYIIG KDTWVEHWPE 360
EDECQDEENQ KQCQDLGAFT ESMVVFGCPN 390œñç [?]
[?]ARNDBCQEZGHILKMFPSTWYVXIQIQ[?]PRT[?] [?] [?]€8 [?]NCBI/XP_001724196
2009-06-10[?]1[?]390 [?] €8 HCTD [?]€[?] [?]V3.3[?]GELS[?]homo
sapiensÿ4
60
 120
 180
 240
 300
 360
 420
 480
 540
 600
 660
 720
 780
 782[?][?] [?]
[?]ARNDBCQEZGHILKMFPSTWYVX[?] [?]PRT[?] [?] [?]€; [?]Swiss-Prot/P06396
1988-01-01[?]1[?]782 [?] €; <tR [?]€[?] [?]V3.3
C9 complement[?]homo sapiensÿ [?]MSACRSFAVA ICILEISILT AQYTTSYDPE LTESSGSASH IDCRMSPWSE
WSQCDPCLRQ 60
MFRSRSIEVF GQFNGKRCTD AVGDRRQCVP TEPCEDAEDD CGNDFQCSTG RCIKMRLRCN 120
GDNDCGDFSD EDDCESEPRP PCRDRVVEES ELARTAGYGI NILGMDPLST PFDNEFYNGL 180
CNRDRDGNTL TYYRRPWNVA SLIYETKGEK NFRTEHYEEQ IEAFKSIIQE KTSNFNAAIS 240
LKFTPTETNK AEQCCEETAS SISLHGKGSF RFSYSKNETY QLFLSYSSKK EKMFLHVKGE 300
IHLGRFVMRN RDVVLTTTFV DDIKALPTTY EKGEYFAFLE TYGTHYSSSG SLGGLYELIY 360
VLDKASMKRK GVELKDIKRC LGYHLDVSLA FSEISVGAEF NKDDCVKRGE GRAVNITSEN 420
LIDDVVSLIR GGTRKYAFEL KEKLLRGTVI DVTDFVNWAS SINDAPVLIS QKLSPIYNLV 480
PVKMKNAHLK KQNLERAIED YINEFSVRKC HTCQNGGTVI LMDGKCLCAC PFKFEGIACE 540
ISKQKISEGL PALEFPNEK 559PN S[?]
[?]ARNDBCQEZGHILKMFPSTWYVXGDFF[?]PRT[?] [?] [?]€> [?]Swiss-Prot/P02748
1996-02-01[?]1[?]559 [?] €> LNFL [?]€[?] [?]V3.3[?]PZP[?]homo
sapiensÿf[?]MRKDRLLHLC LVLLLILLSA SDSNSTEPQY MVLVPSLLHT EAPKKGCVLL SHLNETVTVS
60
ASLESGRENR SLFTDLVAEK DLFHCVSFTL PRISASSEVA FLSIQIKGPT QDFRKRNTVL 120
VLNTQSLVFV QTDKPMYKPG QTVRFRVVSV DENFRPRNEL IPLIYLENPR RNRIAQWQSL 180
KLEAGINQLS FPLSSEPIQG SYRVVVQTES GGRIQHPFTV EEFVLPKFEV KVQVPKIISI 240
MDEKVNITVC GEYTYGKPVP GLATVSLCRK LSRVLNCDKQ EVCEEFSQQL NSNGCITQQV 300
HTKMLQITNT GFEMKLRVEA RIREEGTDLE VTANRISEIT NIVSKLKFVK VDSHFRQGIP 360
FFAQVLLVDG KGVPIPNKLF FISVNDANYY SNATTNEQGL AQFSINTTSI SVNKLFVRVF 420
TVHPNLCFHY SWVAEDHQGA QHTANRVFSL SGSYIHLEPV AGTLPCGHTE TITAHYTLNR 480

QAMGELSELS FHYLIMAKGV IVRSGTHTLP VESGDMKGSF ALSFPVESDV APIARMFIFA 540
ILPDGEVVGD SEKFEIENCL ANKVDLSFSP AQSPPASHAH LQVAAAPQSL CALRAVDQSV 600
LLMKPEAELS VSSVYNLLTV KDLTNFPDNV DQQEEEQGHC PRPFFIHNGA IYVPLSSNEA 660
DIYSFLKGMG LKVFTNSKIR KPKSCSVIPS VSAGAVGQGY YGAGLGVVER PYVPQLGTYN 720
VIPLNNEQSS GPVPETVRSY FPETWIWELV AVNSSGVAEV GVTVPDTITE WKAGAFCLSE 780
DAGLGISSTA SLRAFQPFFV ELTMPYSVIR GEVFTLKATV LNYLPKCIRV SVQLKASPAF 840
LASQNTKGEE SYCICGNERQ TLSWTVTPKT LGNVNFSVSA EAMQSLELCG NEVVEVPEIK 900
RKDTVIKTLL VEAEGIEQEK TFSSMTCASG ANVSEQLSLK LPSNVVKESA RASFSVLGDI 960
LGSAMQNIQN LLQMPYGCGE QNMVLFAPNI YVLNYLNETQ QLTQEIKAKA VGYLITGYQR 1020
QLNYKHQDGS YSTFGERYGR NQGNTWLTAF VLKTFAQARS YIFIDEAHIT QSLTWLSQMQ 1080
KDNGCFRSSG SLLNNAIKGG VEDEATLSAY VTIALLEIPL PVTNPIVRNA LFCLESAWNV 1140
AKEGTHGSHV YTKALLAYAF SLLGKQNQNR EILNSLDKEA VKEDNLVHWE RPQRPKAPVG 1200
HLYQTQAPSA EVEMTSYVLL AYLTAQPAPT SGDLTSATNI VKWIMKQQNA QGGFSSTQDT 1260
VVALHALSRY GAATFTRTEK TAQVTVQDSQ TFSTNFQVDN NNLLLLQQIS LPELPGEYVI 1320
TVTGERCVYL QTSMKYNILP EKEDSPFALK VQTVPQTCDG HKAHTSFQIS LTISYTGNRP 1380
ASNMVIVDVK MVSGFIPLKP TVKMLERSSS VSRTEVSNNH VLIYVEQVTN QTLSFSFMVL 1440
QDIPVGDLKP AIVKVYDYYE TDESVVAEYI APCSTDTEHG NV 1482 [?] [?]
[?]ARNDBCQEZGHILKMFPSTWYVX[?] [?]PRT[?] [?] [?]€A [?]Swiss-Prot/P20742
2009-12-15[?]1[?]1482 [?] €A 18 [?] ÿÿ[?]
CorgApplicant[?] [?]15385 NE 90th Street[?]Redmond
Washington[?]USA[?]98052[?]425-2844390 x321[?]206-2844396
Onconome Inc.[?] ÿÿ[?] [?] CIndivApplicant[?] [?]114 N 200th street Shoreline
Washington[?]USA[?]98133[?]206-5464963 [?]Eva [?]Aw F€[?] [?]114 N 200th street Shoreline
Washington[?]USA[?]98133[?]206-5464963 [?]eawdna@yahoo.com[?]Eva [?]Aw

## Claims

1. A method for detecting colorectal cancer comprising:
determining the presence, an amount and/or an expression level of at least two biomarkers from a biological sample obtained from a subject, wherein one of the biomarkers is Gelsolin (GSN);
comparing the presence, the amount and/or the expression level of the at least two biomarkers with the presence, corresponding amount and/or corresponding expression levels of the at least two biomarkers in a normal control sample; and
based on the comparison, determining the likelihood that the subject has colorectal cancer.

2. The method of claim 1, wherein the biological sample is selected from the group consisting of whole blood, blood plasma, serum, urine, tissue sample, cell sample, and tumor sample.

3. The method of claim 1 or 2, wherein the biological sample is serum.

4. The method of any one of claims 1 to 3, wherein the expression level of the at least two biomarkers in the biological sample from the subject shows a difference as compared with the expression level in the normal control sample, and wherein the difference detects the presence of colorectal cancer in the subject.

5. The method of claim 4,
wherein the difference is increased and is at least 1.05 fold greater in the subject as compared with the normal control sample, and wherein the difference detects the presence of colorectal cancer in the subject, or
wherein the difference is decreased and is at least 0.9 fold less than the normal control sample, and wherein the difference detects the presence of colorectal cancer in the subject.

6. The method of any one of claims 1 to 5, wherein the at least two biomarkers further include any one or more of C2 complement
(C2), C9 Complement Component (C9), alpha-1-acid glycoprotein 1 (ORM1), Pregnancy zone protein, (PZP), C-reactive protein (CRP), Complement factor H-related protein 1 (CFHR1), Plasma serine protease inhibitor (SERPINA3), Hyaluronan-binding protein 2 (HABP2), Beta-Ala-His dipeptidase (CNDP1), Complement factor H-related protein (CFHR2), Serum amyloid A protein (SAA),
LOC653879 similar to complement C3 (LOC653879), Vitamin K-dependent protein Z (PROZ), Serum paraoxonase/lactonase 3 (PON3), Retinoic
acid receptor responder protein 2 (RARRES2}, Gamma-glutamyl hydrolase
(GGH), Proteoglycan-4 (PRG4), Cell surface glycoprotein MUC18
(MCAM) or FN1 Isoform 8 of fibronectin (FN1).

7. The method of claim 4,
wherein the difference is determined by mass spectrometry, immunohistochemistry, ELISA or Western blotting, or
wherein the difference is determined by relative quantitative Multiple Reaction Monitoring (MRM-MS)-LC/MS/MS, or
wherein the difference is determined by quantitative Multiple Reaction Monitoring (MRM-MS)-LC/MS/MS using stable isotope-labeled peptides corresponding to peptides derived from the at least two biomarkers, mentioned in claim 1.

8. The method of any one of claims 1 to 7, wherein the at least two biomarkers include nucleic acid biomarkers.

9. The method of claim 8, further comprising comparing the presence or absence, or the amount or concentration, of one or more nucleic acid biomarkers in the biological sample with the presence or absence,
or the amount or concentration, of one or more nucleic acid biomarkers in the normal control sample.

10. The method of claim 9, wherein the detecting of the presence or absence, or the amount or concentration of, one or more nucleic acid biomarkers is carried out by RT-PCR.

11. The method of any one of the above claims, wherein the at least two biomarkers further comprises CEA.

12. The method of any one of the above claims, wherein the at least two biomarkers further comprises CEA and carbohydrate antigen 19-9.

13. Use of a kit for detecting colorectal cancer by comparing the presence or absence, or the amount or concentration, of at least two biomarkers listed in Table 1 including Gelsolin (GSN) in the biological sample, wherein the biological sample is a biological fluid, with the presence or absence, or the amount or concentration, of said at least two biomarkers in the normal control sample, wherein the kit comprises antibodies, or antibody fragments, which selectively bind to the biomarkers, and instructions for use.

14. Use of a kit for detecting colorectal cancer by comparing the presence or absence, or the amount or concentration, of at least two nucleic acids encoding at least two protein biomarkers listed in Table 1 including a nucleic acid encoding Gelsolin (GSN) in a biological sample, wherein the biological sample is a biological fluid, with the presence or absence, or the amount or concentration, of the at least two nucleic acids encoding at least two protein biomarkers in the normal control sample, wherein the kit comprises an mRNA extracting buffer, at least one reverse transcriptase enzyme, at least one pair of primer suitable for amplifying the nucleic acids encoding the at least two protein biomarkers including Gelsolin (GSN), and instructions for use.

15. Use of a kit for detecting colorectal cancer by comparing the amount or concentration, of at least two biomarkers listed in Table 1 including Gelsolin (GSN), wherein the biological sample is a biological fluid, with the amount or concentration, of the at least two biomarkers in the normal control sample, wherein the kit comprises isotopically labeled peptides corresponding to peptides derived from the at least two biomarkers including Gelsolin (GSN), an internal standard, a set of calibrators, and instructions for use.
